(19) Europäisches Patentamt
European Patent Office
Office européen des brevets

(11) EP 2 540 737 B1

(12) FASCICULE DE BREVET EUROPEEN

(45) Date de publication et mention de la délivrance du brevet:
**01.07.2015 Bulletin 2015/27**

(51) Int Cl.:
*C07K 14/16* (2006.01) *A61K 38/00* (2006.01)
*A61P 35/00* (2006.01)

(21) Numéro de dépôt: **12185309.7**

(22) Date de dépôt: **27.04.2007**

# (54) Utilisation de ligands synthétiques multivalents de la nucléoline de surface pour le traitement de l'inflammation

Einsatz von multivalenten synthetischen Liganden des Oberflächennukleolins zur Entzündungsbehandlung

Use of multivalent synthetic ligands of surface nucleolin for the treatment of inflammation

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **27.04.2006 FR 0603813**

(43) Date de publication de la demande:
**02.01.2013 Bulletin 2013/01**

(62) Numéro(s) de document de la (des) demande(s) initiale(s) en application de l'article 76 CBE:
**07731382.3 / 2 013 232**

(73) Titulaire: **Centre National de la Recherche Scientifique (CNRS)**
**75016 Paris (FR)**

(72) Inventeurs:
- **Courty, José**
  **94440 Villecresnes (FR)**
- **Hovanessian, Ara**
  **Ain Saadeh (LB)**
- **Briand, Jean Paul**
  **67000 Strasbourg (FR)**
- **Guichard, Gilles**
  **67202 Wolfisheim (FR)**
- **Hamma, Yamina**
  **94270 Le Kremlin Bicetre (FR)**

(74) Mandataire: **Regimbeau**
**20, rue de Chazelles**
**75847 Paris Cedex 17 (FR)**

(56) Documents cités:
**WO-A-95/29190**

- **NISOLE SEBASTIEN ET AL: "The anti-HIV pentameric pseudopeptide HB-19 binds the C-terminal end of nucleolin and prevents anchorage of virus particles in the plasma membrane of target cells", JOURNAL OF BIOLOGICAL CHEMISTRY, vol. 277, no. 23, 7 juin 2002 (2002-06-07) , pages 20877-20886, XP002414667, ISSN: 0021-9258**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

SEQUENCE LISTING

<110> CENTRE NATIONAL DE LA RECHERCHE SCIENTIFIQUE (CNRS)

<120> Utilisation de ligands synthétiques multivalents de la nucléoline de surface pour le traitement de l'inflammation

<130> 70190D24262

<140> PCT/FR2007/000730
<141> 2007-04-27

<150> FR0603813
<151> 2006-04-27

<160> 21

<170> PatentIn version 3.5

<210> 1
<211> 10
<212> PRT
<213> artificial sequence

<220>
<223> Peptide synthétique

<400> 1

```
Lys Lys Lys Gly Pro Lys Glu Lys Gly Cys
1               5                   10
```

<210> 2
<211> 6
<212> PRT
<213> artificial sequence

<220>
<223> Peptide synthétique

<400> 2

```
Lys Lys Lys Lys Gly Cys
1               5
```

<210> 3
<211> 12
<212> PRT
<213> artificial sequence

<220>
<223> Peptide synthétique

<400> 3

```
Lys Lys Lys Lys Gly Pro Lys Lys Lys Lys Gly Ala
1               5                   10
```

<210> 4
<211> 9

<212> PRT
<213> artificial sequence

<220>
<223> Peptide synthétique

<220>
<221> MOD_RES
<222> (9)..(9)
<223> Xaa représente le (2S)-2-aminohexanamide

<400> 4

```
Lys Lys Lys Gly Pro Lys Glu Lys Xaa
1               5
```

<210> 5
<211> 11
<212> PRT
<213> artificial sequence

<220>
<223> Peptide synthétique

<220>
<221> BINDING
<222> (1)..(1)
<223> Lysinyl proline

<220>
<221> BINDING
<222> (3)..(3)
<223> N6-Arg-Pro-Lysinyl

<220>
<221> BINDING
<222> (5)..(5)
<223> N6-Arg-Pro-Lysinyl

<220>
<221> BINDING
<222> (8)..(8)
<223> N6-Arg-Pro-Lysinyl

<220>
<221> BINDING
<222> (10)..(10)
<223> N6-Arg-Pro-Lysinyl

<220>
<221> MOD_RES
<222> (11)..(11)
<223> Xaa représente le (2S)-2-aminohexanamide

<400> 5

Pro Arg Lys Lys Lys Gly Pro Lys Glu Lys Xaa
1 5 10

<210> 6
<211> 4
<212> PRT
<213> artificial sequence

<220>
<223> Peptide synthétique

<220>
<221> MOD_RES
<222> (1)..(1)
<223> Aib (2-aminoisobutyric acid)

<220>
<221> MOD_RES
<222> (3)..(3)
<223> Aib (2-aminoisobutyric acid)

<400> 6

Xaa Lys Xaa Gly
1

<210> 7
<211> 3
<212> PRT
<213> artificial sequence

<220>
<223> Peptide synthétique

<220>
<221> MOD_RES
<222> (2)..(2)
<223> Aib (2-aminoisobutyric acid)

<400> 7

Lys Xaa Gly
1

<210> 8
<211> 20
<212> PRT
<213> artificial sequence

<220>
<223> Peptide synthétique

<220>

<221> MOD_RES
<222> (1)..(1)
<223> Aib (2-aminoisobutyric acid)

<220>
<221> MOD_RES
<222> (3)..(3)
<223> Aib (2-aminoisobutyric acid)

<220>
<221> MOD_RES
<222> (5)..(5)
<223> Aib (2-aminoisobutyric acid)

<220>
<221> MOD_RES
<222> (7)..(7)
<223> Aib (2-aminoisobutyric acid)

<220>
<221> MOD_RES
<222> (9)..(9)
<223> Aib (2-aminoisobutyric acid)

<220>
<221> MOD_RES
<222> (11)..(11)
<223> Aib (2-aminoisobutyric acid)

<220>
<221> MOD_RES
<222> (13)..(13)
<223> Aib (2-aminoisobutyric acid)

<220>
<221> MOD_RES
<222> (15)..(15)
<223> Aib (2-aminoisobutyric acid)

<220>
<221> MOD_RES
<222> (17)..(17)
<223> Aib (2-aminoisobutyric acid)

<220>
<221> MOD_RES
<222> (19)..(19)
<223> Aib (2-aminoisobutyric acid)

<400> 8

```
Xaa Lys Xaa Gly Xaa Lys Xaa Gly Xaa Lys Xaa Gly Xaa Lys Xaa Gly
1               5                   10                  15

Xaa Lys Xaa Gly
            20
```

<210> 9
<211> 15
<212> PRT
<213> artificial sequence

<220>
<223> Peptide synthétique

<220>
<221> MOD_RES
<222> (2)..(2)
<223> Aib (2-aminoisobutyric acid)

<220>
<221> MOD_RES
<222> (5)..(5)
<223> Aib (2-aminoisobutyric acid)

<220>
<221> MOD_RES
<222> (8)..(8)
<223> Aib (2-aminoisobutyric acid)

<220>
<221> MOD_RES
<222> (11)..(11)
<223> Aib (2-aminoisobutyric acid)

<220>
<221> MOD_RES
<222> (14)..(14)
<223> Aib (2-aminoisobutyric acid)

<400> 9

**Lys Xaa Gly Lys Xaa Gly Lys Xaa Gly Lys Xaa Gly Lys Xaa Gly**
**1 5 10 15**

<210> 10
<211> 20
<212> PRT
<213> artificial sequence

<220>
<223> Peptide synthétique

<220>
<221> MOD_RES
<222> (1)..(1)
<223> Aib (2-aminoisobutyric acid)

<220>
<221> BINDING
<222> (2)..(2)
<223> N6-Arg-Pro-Lysinyl

<220>

<221> MOD_RES
<222> (3)..(3)
<223> Aib (2-aminoisobutyric acid)

<220>
<221> MOD_RES
<222> (5)..(5)
<223> Aib (2-aminoisobutyric acid)

<220>
<221> BINDING
<222> (6)..(6)
<223> N6-Arg-Pro-Lysinyl

<220>
<221> MOD_RES
<222> (7)..(7)
<223> Aib (2-aminoisobutyric acid)

<220>
<221> MOD_RES
<222> (9)..(9)
<223> Aib (2-aminoisobutyric acid)

<220>
<221> BINDING
<222> (10)..(10)
<223> N6-Arg-Pro-Lysinyl

<220>
<221> MOD_RES
<222> (11)..(11)
<223> Aib (2-aminoisobutyric acid)

<220>
<221> MOD_RES
<222> (13)..(13)
<223> Aib (2-aminoisobutyric acid)

<220>
<221> BINDING
<222> (14)..(14)
<223> N6-Arg-Pro-Lysinyl

<220>
<221> MOD_RES
<222> (15)..(15)
<223> Aib (2-aminoisobutyric acid)

<220>
<221> MOD_RES
<222> (17)..(17)
<223> Aib (2-aminoisobutyric acid)

<220>
<221> BINDING
<222> (18)..(18)
<223> N6-Arg-Pro-Lysinyl

<220>
<221> MOD_RES
<222> (19)..(19)
<223> Aib (2-aminoisobutyric acid)

<400> 10

```
Xaa Lys Xaa Gly Xaa Lys Xaa Gly Xaa Lys Xaa Gly Xaa Lys Xaa Gly
1               5                   10                  15

Xaa Lys Xaa Gly
            20
```

<210> 11
<211> 15
<212> PRT
<213> artificial sequence

<220>
<223> Peptide synthétique

<220>
<221> BINDING
<222> (1)..(1)
<223> N6-Arg-Pro-Lysinyl

<220>
<221> MOD_RES
<222> (2)..(2)
<223> Aib 2-aminoisobutyric acid)

<220>
<221> BINDING
<222> (4)..(4)
<223> N6-Arg-Pro-Lysinyl

<220>
<221> MOD_RES
<222> (5)..(5)
<223> Aib 2-aminoisobutyric acid)

<220>
<221> BINDING
<222> (7)..(7)
<223> N6-Arg-Pro-Lysinyl

<220>
<221> MOD_RES
<222> (8)..(8)
<223> Aib 2-aminoisobutyric acid)

<220>
<221> BINDING
<222> (10)..(10)
<223> N6-Arg-Pro-Lysinyl

<220>

<221> MOD_RES
<222> (11)..(11)
<223> Aib 2-aminoisobutyric acid)

<220>
<221> BINDING
<222> (13)..(13)
<223> N6-Arg-Pro-Lysinyl

<220>
<221> MOD_RES
<222> (14)..(14)
<223> Aib 2-aminoisobutyric acid)

<400> 11

```
Lys Xaa Gly Lys Xaa Gly Lys Xaa Gly Lys Xaa Gly Lys Xaa Gly
1               5                   10                  15
```

<210> 12
<211> 5
<212> PRT
<213> artificial sequence

<220>
<223> Peptide synthétique

<400> 12

```
Lys Ala Lys Pro Gly
1               5
```

<210> 13
<211> 15
<212> PRT
<213> artificial sequence

<220>
<223> Peptide synthétique

<220>
<221> MOD_RES
<222> (1)..(1)
<223> Acetyl lysine

<220>
<221> MOD_RES
<222> (15)..(15)
<223> Amide glycine

<400> 13

```
Lys Ala Lys Pro Gly Lys Ala Lys Pro Gly Lys Ala Lys Pro Gly
1               5                   10                  15
```

<210> 14

<211> 24
<212> PRT
<213> artificial sequence

<220>
<223> Peptide synthétique

<220>
<221> MOD_RES
<222> (1)..(1)
<223> Aib (2-aminoisobutyric acid)

<220>
<221> MOD_RES
<222> (3)..(3)
<223> Aib (2-aminoisobutyric acid)

<220>
<221> MOD_RES
<222> (5)..(5)
<223> Aib (2-aminoisobutyric acid)

<220>
<221> MOD_RES
<222> (7)..(7)
<223> Aib (2-aminoisobutyric acid)

<220>
<221> MOD_RES
<222> (9)..(9)
<223> Aib (2-aminoisobutyric acid)

<220>
<221> VARIANT
<222> (11)..(11)
<223> Aib (2-aminoisobutyric acid)

<220>
<221> MOD_RES
<222> (13)..(13)
<223> Aib (2-aminoisobutyric acid)

<220>
<221> MOD_RES
<222> (15)..(15)
<223> Aib (2-aminoisobutyric acid)

<220>
<221> MOD_RES
<222> (16)..(16)
<223> Aib (2-aminoisobutyric acid)

<220>
<221> MOD_RES
<222> (17)..(17)
<223> Aib (2-aminoisobutyric acid)

<220>

<221> MOD_RES
<222> (19)..(19)
<223> Aib (2-aminoisobutyric acid)

<220>
<221> MOD_RES
<222> (21)..(21)
<223> Aib (2-aminoisobutyric acid)

<220>
<221> MOD_RES
<222> (23)..(23)
<223> Aib (2-aminoisobutyric acid)

<220>
<221> MOD_RES
<222> (24)..(24)
<223> Amide glycine

<400> 14

```
Xaa Lys Xaa Gly Xaa Lys Xaa Gly Xaa Lys Xaa Gly Xaa Lys Xaa Gly
1               5                   10                  15

Xaa Lys Xaa Gly Xaa Lys Xaa Gly
            20
```

<210> 15
<211> 18
<212> PRT
<213> artificial sequence

<220>
<223> Peptide synthétique

<220>
<221> MOD_RES
<222> (1)..(1)
<223> Acetyl lysine

<220>
<221> MOD_RES
<222> (2)..(2)
<223> Aib (2-aminoisobutyric acid)

<220>
<221> MOD_RES
<222> (5)..(5)
<223> Aib (2-aminoisobutyric acid)

<220>
<221> MOD_RES
<222> (8)..(8)
<223> Aib (2-aminoisobutyric acid)

<220>
<221> MOD_RES

<222> (11)..(11)
<223> Aib (2-aminoisobutyric acid)

<220>
<221> MOD_RES
<222> (14)..(14)
<223> Aib (2-aminoisobutyric acid)

<220>
<221> MOD_RES
<222> (17)..(17)
<223> Aib (2-aminoisobutyric acid)

<220>
<221> MOD_RES
<222> (18)..(18)
<223> Amide glycine

<400> 15

```
Lys Xaa Gly Lys Xaa Gly Lys Xaa Gly Lys Xaa Gly Lys Xaa Gly Lys
1               5                   10                  15

Xaa Gly
```

<210> 16
<211> 18
<212> PRT
<213> artificial sequence

<220>
<223> Peptide synthétique

<220>
<221> MOD_RES
<222> (1)..(1)
<223> Acetyl lysine

<220>
<221> BINDING
<222> (1)..(1)
<223> N6-Arg-Pro-Lysinyl

<220>
<221> MOD_RES
<222> (2)..(2)
<223> Aib (2-aminoisobutyric acid)

<220>
<221> BINDING
<222> (4)..(4)
<223> LYS-X-PRO-ARG-LYS. X represents the (-CH2NH-) binding

<220>
<221> MOD_RES
<222> (5)..(5)
<223> Aib (2-aminoisobutyric acid)

<220>
<221> BINDING
<222> (7)..(7)
<223> N6-Arg-Pro-Lysinyl

<220>
<221> MOD_RES
<222> (8)..(8)
<223> Aib (2-aminoisobutyric acid)

<220>
<221> BINDING
<222> (10)..(10)
<223> N6-Arg-Pro-Lysinyl

<220>
<221> VARIANT
<222> (11)..(11)
<223> Aib (2-aminoisobutyric acid)

<220>
<221> BINDING
<222> (13)..(13)
<223> N6-Arg-Pro-Lysinyl

<220>
<221> MOD_RES
<222> (14)..(14)
<223> Aib (2-aminoisobutyric acid)

<220>
<221> BINDING
<222> (16)..(16)
<223> N6-Arg-Pro-Lysinyl

<220>
<221> MOD_RES
<222> (17)..(17)
<223> Aib (2-aminoisobutyric acid)

<220>
<221> MOD_RES
<222> (18)..(18)
<223> Amide glycine

<400> 16

**Lys Xaa Gly Lys Xaa Gly Lys Xaa Gly Lys Xaa Gly Lys Xaa Gly Lys**
**1 5 10 15**

**Xaa Gly**

<210> 17
<211> 15
<212> PRT
<213> artificial sequence

<220>
<223> Peptide synthétique

<220>
<221> MOD_RES
<222> (1)..(1)
<223> Acetyl lysine

<220>
<221> BINDING
<222> (1)..(1)
<223> N6-Arg-Pro-Lysinyl

<220>
<221> BINDING
<222> (3).. (3)
<223> N6-Arg-Pro-Lysinyl

<220>
<221> BINDING
<222> (6)..(6)
<223> N6-Arg-Pro-Lysinyl

<220>
<221> BINDING
<222> (8)..(8)
<223> N6-Arg-Pro-Lysinyl

<220>
<221> BINDING
<222> (11)..(11)
<223> N6-Arg-Pro-Lysinyl

<220>
<221> BINDING
<222> (13)..(13)
<223> N6-Arg-Pro-Lysinyl

<220>
<221> MOD_RES
<222> (15)..(15)
<223> Amide glycine

<400> 17

```
Lys Ala Lys Pro Gly Lys Ala Lys Pro Gly Lys Ala Lys Pro Gly
1               5                   10                  15
```

<210> 18
<211> 20
<212> PRT
<213> artificial sequence

<220>
<223> Peptide synthétique

<220>

<221> MOD_RES
<222> (1)..(1)
<223> Aib (2-aminoisobutyric acid)

<220>
<221> MOD_RES
<222> (1)..(1)
<223> Acetyl lysine

<220>
<221> MOD_RES
<222> (3)..(3)
<223> Aib (2-aminoisobutyric acid)

<220>
<221> MOD_RES
<222> (5)..(5)
<223> Aib (2-aminoisobutyric acid)

<220>
<221> MOD_RES
<222> (7)..(7)
<223> Aib (2-aminoisobutyric acid)

<220>
<221> MOD_RES
<222> (9)..(9)
<223> Aib (2-aminoisobutyric acid)

<220>
<221> MOD_RES
<222> (11)..(11)
<223> Aib (2-aminoisobutyric acid)

<220>
<221> MOD_RES
<222> (13)..(13)
<223> Aib (2-aminoisobutyric acid)

<220>
<221> MOD_RES
<222> (15)..(15)
<223> Aib (2-aminoisobutyric acid)

<220>
<221> MOD_RES
<222> (17)..(17)
<223> Aib (2-aminoisobutyric acid)

<220>
<221> MOD_RES
<222> (19)..(19)
<223> Aib (2-aminoisobutyric acid)

<220>
<221> MOD_RES
<222> (20)..(20)
<223> Amide glycine

<400> 18

```
Xaa Lys Xaa Gly Xaa Lys Xaa Gly Xaa Lys Xaa Gly Xaa Lys Xaa Gly
1               5                   10                  15

Xaa Lys Xaa Gly
            20
```

<210> 19
<211> 15
<212> PRT
<213> artificial sequence

<220>
<223> Peptide synthétique

<220>
<221> MOD_RES
<222> (1)..(1)
<223> Acetyl lysine

<220>
<221> MOD_RES
<222> (2)..(2)
<223> Aib (2-aminoisobutyric acid)

<220>
<221> MOD_RES
<222> (5)..(5)
<223> Aib (2-aminoisobutyric acid)

<220>
<221> MOD_RES
<222> (8)..(8)
<223> Aib (2-aminoisobutyric acid)

<220>
<221> MOD_RES
<222> (11)..(11)
<223> Aib (2-aminoisobutyric acid)

<220>
<221> MOD_RES
<222> (14)..(14)
<223> Aib (2-aminoisobutyric acid)

<220>
<221> MOD_RES
<222> (15)..(15)
<223> Amide glycine

<400> 19

```
Lys Xaa Gly Lys Xaa Gly Lys Xaa Gly Lys Xaa Gly Lys Xaa Gly
1               5                   10                  15
```

<210> 20
<211> 20
<212> PRT
<213> artificial sequence

<220>
<223> Peptide synthétique

<220>
<221> MOD_RES
<222> (1)..(1)
<223> Aib (2-aminoisobutyric acid)

<220>
<221> MOD_RES
<222> (1)..(1)
<223> Acetyl lysine

<220>
<221> BINDING
<222> (2)..(2)
<223> N6-Arg-Pro-Lysinyl

<220>
<221> MOD_RES
<222> (3)..(3)
<223> Aib (2-aminoisobutyric acid)

<220>
<221> MOD_RES
<222> (5)..(5)
<223> Aib (2-aminoisobutyric acid)

<220>
<221> BINDING
<222> (6)..(6)
<223> N6-Arg-Pro-Lysinyl

<220>
<221> MOD_RES
<222> (7)..(7)
<223> Aib (2-aminoisobutyric acid)

<220>
<221> MOD_RES
<222> (9)..(9)
<223> Aib (2-aminoisobutyric acid)

<220>
<221> BINDING
<222> (10)..(10)
<223> N6-Arg-Pro-Lysinyl

<220>
<221> MOD_RES
<222> (11)..(11)
<223> Aib (2-aminoisobutyric acid)

<220>
<221> MOD_RES
<222> (13)..(13)
<223> Aib (2-aminoisobutyric acid)

<220>
<221> BINDING
<222> (14)..(14)
<223> N6-Arg-Pro-Lysinyl

<220>
<221> MOD_RES
<222> (15)..(15)
<223> Aib (2-aminoisobutyric acid)

<220>
<221> MOD_RES
<222> (17)..(17)
<223> Aib (2-aminoisobutyric acid)

<220>
<221> BINDING
<222> (18)..(18)
<223> N6-Arg-Pro-Lysinyl

<220>
<221> MOD_RES
<222> (19)..(19)
<223> Aib (2-aminoisobutyric acid)

<220>
<221> MOD_RES
<222> (20)..(20)
<223> Amide glycine

<400> 20

```
Xaa Lys Xaa Gly Xaa Lys Xaa Gly Xaa Lys Xaa Gly Xaa Lys Xaa Gly
1               5                   10                  15

Xaa Lys Xaa Gly
            20
```

<210> 21
<211> 15
<212> PRT
<213> artificial sequence

<220>
<223> Peptide synthétique

<220>
<221> MOD_RES
<222> (1)..(1)
<223> Acetyl lysine

<220>
<221> BINDING
<222> (1)..(1)
<223> N6-Arg-Pro-Lysinyl

<220>
<221> MOD_RES
<222> (2)..(2)
<223> Aib (2-aminoisobutyric acid)

<220>
<221> BINDING
<222> (4)..(4)
<223> N6-Arg-Pro-Lysinyl

<220>
<221> MOD_RES
<222> (5)..(5)
<223> Aib (2-aminoisobutyric acid)

<220>
<221> BINDING
<222> (7)..(7)
<223> N6-Arg-Pro-Lysinyl

<220>
<221> MOD_RES
<222> (8)..(8)
<223> Aib (2-aminoisobutyric acid)

<220>
<221> BINDING
<222> (10)..(10)
<223> N6-Arg-Pro-Lysinyl

<220>
<221> MOD_RES
<222> (11)..(11)
<223> Aib (2-aminoisobutyric acid)

<220>
<221> BINDING
<222> (13)..(13)
<223> N6-Arg-Pro-Lysinyl

<220>
<221> MOD_RES
<222> (14)..(14)
<223> Aib (2-aminoisobutyric acid)

<220>
<221> MOD_RES
<222> (15)..(15)
<223> Amide glycine

<400> 21

```
Lys Xaa Gly Lys Xaa Gly Lys Xaa Gly Lys Xaa Gly Lys Xaa Gly
1           5                       10                  15
```

## Description

La présente invention concerne l'utilisation d'un composé synthétique multivalent comprenant ou constitué d'un support sur lequel sont greffés au moins 3 motifs pseudopeptidiques, ledit composé étant de formule (I) :

$$[(X)_n - Y_1 \overset{\Psi}{-} (Z)_i - Y_2 - (X)_m]_k - \text{Support} \qquad (I)$$

, où chaque X représente indépendamment un acide aminé quelconque ; $Y_1$ et $Y_2$ sont indépendamment choisis parmi les acides aminés à chaîne latérale basique ; Z est choisi parmi une proline, éventuellement substituée en $\gamma$, $\beta$ ou $\delta$ ; un acide aminé naturel ou non N-alkylé ; un acide aminé dialkylé ; un acide aminé dialkylé cyclique ; l'acide pipécolique ou l'un de ses dérivés ; n et i sont indépendamment 0 ou 1 ; m est un entier entre 0 et 3 ; k est un entier supérieur ou égal à 3 ; et $\Psi$ représente une liaison peptidique modifiée, significativement plus résistante à au moins une protéase qu'une liaison peptidique normale, pour la fabrication d'un médicament destiné au traitement d'une maladie inflammatoire.

Il est maintenant bien connu que les maladies chroniques inflammatoires, et notamment les maladies auto-immunes à médiation cellulaire sont en partie induites par des cytokines. Notamment, les résultats obtenus dans différents modèles animaux expérimentaux ont souligné le rôle des cytokines dans la pathogenèse de la maladie (Seko et al-2006). Par exemple, les cytokines pro-inflammatoires telles que le facteur de nécrose tumorale $\alpha$ (TNF-$\alpha$), l'interleukine 1(IL-1), l'IL-6, l'IL-15 et l'IL-18 régulent les réponses immunitaires et inflammatoires chez les patients souffrant de polyarthrite rhumatoïde. En particulier, le TNF-$\alpha$ et l'IL-1$\beta$ favorisent la destruction du cartilage et de la moelle osseuse.

En outre, au cours du processus inflammatoire, l'endothélium vasculaire exprime différentes chimiokines et molécules d'adhésion qui participent au recrutement sélectif des leucocytes au niveau du foyer inflammatoire (Rot et al-1993).

IL-8 est une chimiokine C-X-C qui initie l'activation et le recrutement sélectif des leucocytes au niveau des sites tissulaires de l'inflammation. Lorsqu'elle est exprimée à des niveaux élevés, l'IL-8 peut avoir des conséquences pathologiques pour l'organisme. Le Lipopolysaccharide (LPS) et les cytokines pro-inflammatoires telles que le TNF-$\alpha$ et l'IL-1 induisent la sécrétion d' IL-8 par de nombreux types cellulaires, en particulier les cellules endothéliales (Elass et al-2002).

La molécule d'adhésion cellulaire intercellulaire-1 (ICAM-1) est une protéine de type immunoglobuline exprimée à la surface de plusieurs types cellulaires incluant les cellules endothéliales et les cellules impliquées dans la réponse immunitaire. Elle joue un rôle important dans l'adhésion et la migration des leucocytes vers les sites d'inflammation (Kevil et al-2001).

Les cytokines pro-inflammatoires sont également impliquées dans la réponse inflammatoire généralisée (choc sceptique) induite par les infections bactériennes. Le Lipopolysaccharide (LPS) est un composant intégral de la membrane externe des bactéries gram-négatives. Cette molécule immuno-stimulatrice est un facteur qui contribue de façon majoritaire à l'initiation d'une réponse inflammatoire généralisée appelée choc sceptique, qui accompagne souvent une bactériémie gram-négative. Le LPS a notamment comme propriété biologique de stimuler la production de cytokines telles que le TNF-$\alpha$, l'IL-1 et l'IL-6 par les cellules lymphoréticulaires. L'induction de ces cytokines joue un rôle pivot dans le développement du syndrome sceptique, puisque l'administration de TNF-$\alpha$ seul peut conduire à un état sceptique et à la mort, le TNF-$\alpha$ pouvant induire la production d'IL-1 et d'IL-6 in vivo. En outre, dans des modèles animaux, un pré-traitement avec un anticorps anti-TNF-$\alpha$ et un antagoniste du récepteur de l'IL-1 permettent de protéger les animaux contre les effets létaux du LPS (Bucklin et al-1993).

La septie sévère est associée avec une réaction inflammatoire explosive et à des déficiences d'organes, et également souvent associée avec un fort taux de mortalité. Elle peut être consécutive à une infection bactérienne, fongique ou virale. Cette réponse est marquée par la sécrétion séquentielle de cytokines pro-inflammatoires puis anti-inflammatoires. Parmi les cytokines pro-inflammatoires les plus importantes, se trouvent le TNF-$\alpha$ et l'IL-$\beta$. Jusqu'à présent, aucun des essais cliniques impliquant des réactifs anti-LPS ou anti-cytokine n'a été couronné de succès (Karima et al-1999, Zanotti et al-2002).

Les cytokines pro-inflammatoires semblent également jouer un rôle physiopathologique chez les patients souffrant de cardite ou inflammation du coeur, qui se manifeste par une inflammation de l'endocarde (endocardite) ou une inflammation du péricarde (péricardite) ou une inflammation du muscle cardiaque (myocardite). Par exemple, le niveau sérique d'IL-6 est significativement augmenté chez des patients souffrant d'endocardite infectieuse qui peut être induite notamment par une infection par Staphylococcus aureus. De cette façon, un niveau sérique élevé d'IL-6 peut suggérer l'existence d'une péricardite infectieuse et être utilisé comme une aide au diagnostic et au suivi du traitement de la maladie (Alter et al-2002, Shun et al-2005)

Au vu de l'importance du rôle des cytokines pro-inflammatoires telles que le TNF-$\alpha$, l'IL-1, et l'IL-6 dans les maladies inflammatoires, des thérapies anti-cytokines inflammatoires comprenant des réactifs anti-TNF-$\alpha$, anti-IL-1 et anti-IL-6 ont été développées pour le traitement de patients souffrant de maladies inflammatoires (Kannan et al-2005).

Différents essais cliniques impliquant des réactifs anti-cytokine inflammatoire dans le traitement de maladies

inflammatoires chroniques telles que la polyarthrite rhumatoïde et la maladie inflammatoire abdominale ont obtenu un certain succès : Etanercept (protéine de fusion TNF récepteur-P75 Fc), Infliximab (anticorps monoclonal chimérique anti-TNF-$\alpha$ humain), Adalimumab (anticorps monoclonal recombinant humain anti-TNF-$\alpha$ humain) et Anakinra (forme recombinante de l'antagoniste du récepteur IL-1$\beta$ humain)( Karima et al-1999).

Cependant, tous les réactifs anti-cytokine inflammatoire disponibles jusqu'à présent sont des protéines, et souffrent donc des inconvénients associés de façon générale avec les médicaments protéiques. En particulier, ces médicaments ont un coût très élevé et sont difficiles à produire en grande quantité.

Par conséquent, il existe un réel besoin pour des molécules de petit poids moléculaire capables de cibler spécifiquement les voies de synthèse des cytokines pro-inflammatoires.

De façon surprenante, les inventeurs ont trouvé que les composés de formule (I) tels que décrits précédemment possèdent une activité anti-inflammatoire, et notamment inhibent la production de TNF-$\alpha$, d'IL-6 et d'IL-8, ainsi que l'expression d' ICAM-1 par différents types cellulaires stimulés par du LPS.
La nucléoline (voir structure sur la Figure 1A) a initialement été décrite comme une protéine nucléaire présente dans la plupart des cellules eucaryotes. Plus récemment, il a été démontré que, malgré l'absence de domaine trans-membranaire permettant son association à la membrane plasmique, une autre forme moléculaire de cette protéine était présente également à la surface cellulaire (Hovanessian et al-2000, Nisole et al-1999, Ginisty et al-1999, Srivastava et al-1999). Cette nucléoline de surface se trouve associée étroitement aux microfilaments d'actine intracellulaire ; cette association étant selon toute vraisemblance indirecte via un partenaire transmembranaire.

Dans une cellule au repos, la nucléoline est localisée principalement au niveau du nucléole, mais également partiellement dans le cytoplasme et à la surface des cellules. A la suite d'une activation de la prolifération cellulaire, la nucléoline cytoplasmique est transloquée vers la surface membranaire par un mécanisme d'un transport actif, non conventionnel, indépendant du réticulum endoplasmique et de l'appareil de Golgi (Hovanessian et al-2000).

Ainsi, le niveau d'expression de la nucléoline de surface est fortement augmenté suite à l'activation des cellules, et notamment l'activation de la prolifération cellulaire. La nucléoline de surface constitue donc un marqueur des cellules activées, en prolifération. Dans le cas notamment du virus de l'immunodéficience humaine (VIH) qui cible particulièrement les cellules activées, il a été montré que la nucléoline de surface était impliquée dans l'infection de ces cellules par le VIH (Nisole et al-1999, Nisole et al-2002-Exp. Cell Res).

En outre, la nucléoline de surface constitue un récepteur de faible affinité pour différents ligands, et notamment pour plusieurs facteurs de croissance tels que la midkine (MK), l'heparin affin regulatory peptide (HARP ; également connue sous le nom de pleiotrophin : PTN) et la lactoferrine (Said et al-2002, Said et al-2005, Legrand et al-2004).

Le composé multivalent HB19 (voir Figure 2A), synthétisé et décrit par les inventeurs, est un ligand de la nucléoline de surface (Callebaut et al-1997, Callebaut et al-1998, Nisole et al-2000, Nisole et al-2002-J. Biol. Chem) qui interagit au niveau du domaine RGG (voir Figure 1B). Il a été montré que ce composé permettait d'inhiber l'infection des cellules activées par le VIH (Nisole et al-2000) ainsi que la liaison à la nucléoline d'autres ligands naturels (Said et al-2002, Said et al-2005, Legrand et al-2004).

Ces composés de formule (I) sont très intéressants, puisque :

- aucun effet toxique de ces composés n'a été observé par les inventeurs, ni in vitro, ni in vivo. En particulier, aucun effet toxique n'a été observé par les inventeurs, ni sur des cellules cultivées in vitro pendant plusieurs semaines en présence du composé HB19, ni in vivo sur les souris traitées par le composé HB19.
- ces composés sont facilement synthétisables, y compris à l'échelle industrielle, dans des conditions facilement maîtrisables ;
- ces composés possèdent en eux-mêmes une biodisponibilité in vivo suffisante pour ne pas nécessiter le développement de forme galénique particulière. En particulier, la nature pseudopeptidique, et la solubilité élevée en milieu aqueux du composé HB 19 lui permettent de présenter une très bonne biodisponibilité in vivo. En outre, la présence d'une liaison peptidique modifiée (réduite dans le cas du composé HB19) entre la lysine et la proline de chaque motif KPR présenté dans le cas d'HB19 lui confère une bonne résistance aux protéases in vivo et une demi-vie in vivo supérieure à 24h, contrairement à un peptide classique dont la demi-vie in vivo ne dépasse pas une demi heure. De plus, le composé HB19 est complètement soluble en milieu aqueux, ce qui facilite grandement son administration, aucune forme galénique particulière n'étant nécessaire à sa circulation et à son ciblage in vivo.

L'invention concerne donc l'utilisation d'un composé synthétique multivalent de formule (I) :

$$[(X)_n—Y_1\overset{\Psi}{—}(Z)_i—Y_2\text{-}(X)_m]_k—\text{Support} \qquad \text{(I)}$$ ,

où chaque X représente indépendamment un acide aminé quelconque ; $Y_1$ et $Y_2$ sont indépendamment choisis parmi

les acides aminés à chaîne latérale basique ; Z est choisi parmi une proline, éventuellement substituée en γ, β ou δ ; un acide aminé naturel ou non N-alkylé ; un acide aminé dialkylé ; un acide aminé dialkylé cyclique ; l'acide pipécolique ou l'un de ses dérivés ; n et i sont indépendamment 0 ou 1 ; m est un entier entre 0 et 3 ; k est un entier supérieur ou égal à 3 ; et Ψ représente une liaison peptidique modifiée, significativement plus résistante à au moins une protéase qu'une liaison peptidique normale, pour la préparation d'un médicament destiné au traitement des maladies inflammatoires.

Au sens de l'invention, on entend par « support » toute molécule pharmaceutiquement acceptable, c'est-à-dire sans toxicité intrinsèque, sur laquelle au moins 3 motifs pseudopeptidiques de formule (I) peuvent être greffés. Un support acceptable devrait donc posséder une taille suffisante pour permettre de greffer au moins 3 motifs pseudopeptidiques de formule (I), de préférence de 3 à 8 motifs pseudopeptidiques de formule (I). Un tel support acceptable devrait aussi de préférence posséder une taille suffisamment petite pour que lesdits au moins 3, de préférence 3 à 8, motifs pseudopeptidiques de formule (I) puissent venir ensemble interagir au niveau du domaine RGG d'une ou plusieurs molécules de nucléoline. Le support devrait de surcroît ne pas être immunogène.

Un tel support peut notamment être choisi parmi un peptide linéaire ou un peptide cyclique, un peptoïde (oligomère de glycine N-substitué) linéaire ou cyclique, un foldamère (oligomère ou polymère ayant une forte tendance à adopter une conformation compacte bien définie et prévisible en solution), un polymère linéaire ou un dendrimère (macromolécule constituée de monomères qui s'associent selon un processus arborescent autour d'un coeur central plurifonctionnel) sphérique, un sucre, ou une nanoparticule. Avantageusement, ledit support est choisi parmi un peptide linéaire ou cyclique, ou bien un peptoïde linéaire ou cyclique.

L'utilisation d'un peptide linéaire (voir structure de HB19 sur la Figure 2A) permet une synthèse facile du support, et les résultats des inventeurs avec le composé HB19 montrent qu'un tel support permet effectivement de résoudre le problème technique posé dans la présente demande. Un peptide linéaire servant de support dans l'invention peut avantageusement comprendre une proportion de lysine supérieure à 25%. Plus précisément, lorsqu'un peptide linéaire est utilisé comme support dans l'invention, les motifs pseudopeptidiques sont de préférence greffés en position ε d'une lysine. Lorsqu'un peptide linéaire est utilisé comme support dans l'invention, il comprend donc de préférence au moins autant de lysines que le nombre de motifs pseudopeptidiques que l'on souhaite greffer dessus.

Par exemple, un peptide linéaire support peut posséder une séquence choisie parmi KKKGPKEKGC (SEQ ID NO:1), KKKKGC (SEQ ID NO:2), KKKKGPKKKKGA (SEQ ID NO:3) ou KKKGPKEKAhxCONH$_2$ (SEQ ID NO:4), où Ahx représente l'acide amino hexanoïque et CONH$_2$ représente le fait que la fonction acide est remplacée par une fonction amide, AhxCONH$_2$ représentant le (2S)-2-aminohexanamide, ou bien une séquence linéaire constituée de 2 à 4 motifs (KAKPG, SEQ ID NO:12), notamment la séquence AcKAKPGKAKPGKAKPGCONH$_2$ (SEQ ID NO:13, où Ac représente un groupement acétyl $CH_3$-CO-, et CONH$_2$ signifie que la fonction acide COOH de la glycine est remplacée par une fonction amide CONH$_2$). Avantageusement, le peptide linéaire support peut être le peptide KKKGPKEKAhxCONH$_2$ (voir par exemple HB19 sur la Figure 2A, SEQ ID NO:5, qui possède ce peptide linéaire pour support), ou le peptide AcKAKPGKAKPGKAKPGCONH$_2$ (SEQ ID NO:4,, où Ac représente un groupement acétyl $CH_3$-CO- et CONH$_2$ signifie que la fonction acide COOH de la glycine est remplacée par une fonction amide CONH$_2$, voir par exemple Nucant 7 sur la Figure 2F, SEQ ID NO:17, qui possède ce peptide linéaire pour support).

Parmi les peptides linéaires, certains sont connus pour adopter une structure hélicoïdale. Ces peptides linéaires peuvent également être utilisés comme support dans l'invention. De tels supports peptidiques linéaires formant une structure hélicoïdale comprennent notamment des supports constitués d'un nombre entier supérieur ou égal à 3, notamment de 3 à 8, de répétitions de motifs peptidiques de séquence Aib-Lys-Aib-Gly (SEQ ID NO:6) ou Lys-Aib-Gly (SEQ ID NO:7) respectivement, où Aib représente l'acide 2-amino-isobutyrique. Chacun de ces motifs comprenant un seul résidu lysine (Lys), il faudra autant de répétitions de ces motifs qu'on souhaite pouvoir greffer de motifs pseudopeptidiques de formule (I).

Par exemple, pour obtenir un composé pentavalent avec 5 motifs pseudopeptidiques de formule (I), on peut utiliser comme support un peptide linéaire formant une structure hélicoïdale de formule Aib-Lys-Aib-Gly-Aib-Lys-Aib-Gly-Aib-Lys-Aib-Gly-Aib-Lys-Aib-Gly-Aib-Lys-Aib-Gly (SEQ ID NO:8) ou Lys-Aib-Gly-Lys-Aib-Gly-Lys-Aib-Gly-Lys-Aib-Gly-Lys-Aib-Gly (SEQ ID NO:9). Avantageusement, un peptide linéaire formant une structure hélicoïdale de formule dérivée de SEQ ID NO:8 et 9 est utilisé, dont la formule est choisie parmi Ac-Aib-Lys-Aib-Gly-Aib-Lys-Aib-Gly-Aib-Lys-Aib-Gly-Aib-Lys-Aib-Gly-Aib-Lys-Aib-Gly-CONH$_2$ (SEQ ID NO:18, où Ac représente un groupement acétyl $CH_3$-CO- et CONH$_2$ signifie que la fonction acide COOH de la glycine est remplacée par une fonction amide CONH$_2$, voir par exemple Nucant 2 sur la Figure 2C, SEQ ID NO:20, qui possède ce peptide pour support) ou Ac-Lys-Aib-Gly-Lys-Aib-Gly-Lys-Aib-Gly-Lys-Aib-Gly-Lys-Aib-Gly-CONH$_2$ (SEQ ID NO:19, où Ac représente un groupement acétyl $CH_3$-CO- et CONH$_2$ signifie que la fonction acide COOH de la glycine est remplacée par une fonction amide CONH$_2$, voir par exemple Nucant 3 sur la Figure 2D, SEQ ID NO:21, qui possède ce peptide pour support).

Alternativement, pour obtenir un composé hexavalent avec 6 motifs pseudopeptidiques de formule (I), on peut utiliser comme support un peptide linéaire formant une structure hélicoïdale de formule Ac-Aib-Lys-Aib-Gly-Aib-Lys-Aib-Gly-Aib-Lys-Aib-Gly-Aib-Lys-Aib-Gly-Aib-Lys-Aib-Gly-Aib-Lys-Aib-Gly-CONH$_2$ (SEQ ID NO:14, où Ac représente un

groupement $CH_3$-CO- et $CONH_2$ signifie que la fonction acide COOH de la glycine est remplacée par une fonction amide $CONH_2$) ou Ac-Lys-Aib-Gly-Lys-Aib-Gly-Lys-Aib-Gly-Lys-Aib-Gly-Lys-Aib-Gly-Lys-Aib-Gly-$CONH_2$ (SEQ ID NO:15, où Ac représente un groupement $CH_3$-CO- et $CONH_2$ signifie que la fonction acide COOH de la glycine est remplacée par une fonction amide $CONH_2$, voir par exemple Nucant 6 sur la Figure 2E, SEQ ID NO:17, qui possède ce peptide pour support).

Un peptide ou peptoïde cyclique peut également être avantageusement utilisé comme support. Cela permet notamment de restreindre la flexibilité du squelette. Un peptide ou peptoïde cyclique support peut notamment être choisi parmi un hexa-, octa-, déca- ou dodéca- peptide cyclique constitué préférentiellement de résidus d'acides aminés de configuration L (lévogyre) et D (dextrogyre) en alternance (D,L-cyclopeptide) ou bien d'un enchaînement de résidus N-alkyl Glycine (peptoïde cyclique). Un exemple de composé avec comme support un hexapeptide cyclique constitué en alternance de résidus alanine (A) de configuration D et de résidus lysine (K) de configuration L présentant 3 motifs KPR avec une liaison $\Psi$ ($\Psi$ = (-$CH_2$N-)) entre K et P est montré sur la Figure 2B (composé Nucant 01).

Avantageusement, le support d'un composé de formule (I) selon l'invention est support est choisi parmi un hexapeptide cyclique constitué en alternance de résidus alanine (A) de configuration D et de résidus lysine (K) de configuration L, ou un peptide linéaire de séquence SEQ ID NO:1, SEQ ID NO:2, SEQ ID NO:3, SEQ ID NO:4, SEQ ID NO:8, SEQ ID NO:9, SEQ ID NO:13, SEQ ID NO:14, SEQ ID NO:15, SEQ ID NO:18, ou SEQ ID NO:19.

Au sens de l'invention, on entend par « greffés » pour les motifs pseudopeptidiques le fait d'être liés au support par une liaison covalente, soit directement, soit par l'intermédiaire d'un composé espaceur entre les motifs pseudopeptidiques et le support. De ce fait, dans un mode de réalisation particulier, les motifs pseudopeptidiques sont greffés directement sur le support, sans composé espaceur entre eux et le support. Dans un autre mode de réalisation, les motifs pseudopeptidiques sont greffés sur le support par l'intermédiaire d'un espaceur. Des exemples d'espaceurs acceptables comprennent des composés de type éthylène glycol, pipérazine, ou un acide aminé de type acide aminohexanoïque ou beta-alanine.

Dans le cas où le support est un peptide linéaire ou cyclique et où les motifs pseudopeptidiques sont greffés directement sur le peptide, la liaison entre le peptide et les motifs pseudopeptidiques est de préférence réalisée au niveau d'un résidu lysine du peptide support, au niveau d'un groupe amino en position $\alpha$ ou $\varepsilon$, de préférence au niveau du groupe amino en position $\varepsilon$ (sur la chaîne latérale) de la lysine. Ainsi, le greffage direct des motifs pseudopeptidiques sur un support peptidique se fait avantageusement par une liaison amide entre une fonction acide COOH de l'acide aminé en position C-terminale du motif pseudopeptidique et un groupement amino d'un résidu lysine, de préférence le groupement amino en position $\varepsilon$ (sur la chaîne latérale) de la lysine.

Dans les composés selon l'invention, au moins 3 motifs pseudopeptidiques sont greffés sur le support. En effet, les résultats des inventeurs montrent l'importance de la liaison au domaine RGG de la nucléoline (voir Figure 1) pour l'efficacité anti-tumorale exceptionnelle du composé HB19 et des composés dérivés ou analogues. Or la liaison au domaine RGG de la nucléoline est réalisée grâce à la présentation multivalente de plusieurs motifs pseudopeptidiques tels que ceux incorporés dans la formule (I). Pour des composés dont le support est un peptide linéaire de séquence KKKGPKEKGC, KKKKGC, KKKKGPKKKKGA ou KKKGPKEKAhx$CONH_2$, les inventeurs ont montré qu'en dessous de 3 motifs ($k < 3$), l'efficacité de liaison à la nucléoline est moins forte et l'efficacité anti-tumorale en est vraisemblablement amoindrie. Les composés selon l'invention comprennent donc au moins 3 motifs pseudopeptidiques greffés sur un support, k étant donc un nombre entier supérieur ou égal à 3. Les composés selon l'invention présentent donc avantageusement de 3 à 8 motifs pseudopeptidiques ($3 \leq k \leq 8$) greffés sur le support. De plus, les inventeurs ont montré que l'activité était optimale avec 5 ou 6 motifs pseudopeptidiques greffés sur le support ($k = 5$), l'efficacité de liaison à la nucléoline n'augmentant plus pour un nombre plus élevé de motifs pseudopeptidiques. Avantageusement, dans les composés de formule (I), k est donc compris entre 3 et 8, de préférence entre 4 et 7, entre 4 et 6, entre 4 et 5 ou entre 5 et 6. Plus avantageusement encore, dans les composés de formule (I), k vaut 5 ou mieux encore 6.

Au sens de l'invention, on entend par « acide aminé quelconque » tout acide aminé naturel ou de synthèse, éventuellement modifié par la présence d'un ou plusieurs substituants. Plus précisément, on entend par acide aminé un acide alpha aminé répondant à la structure générale :

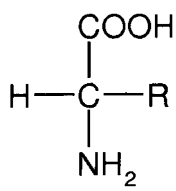
,

où R représente la chaîne latérale de l'acide aminé. Au sens de l'invention, R représente donc la chaîne latérale d'un acide aminé naturel ou non. Par « acide aminé naturel », on entend tout acide aminé pouvant se trouver naturellement

in vivo chez un être vivant. Les acides aminés naturels comprennent donc notamment les acides aminés codés par les ARNm incorporés dans les protéines lors de la traduction, mais également d'autres acides aminés trouvés naturellement in vivo pouvant être un produit ou un sous-produit d'un processus métabolique, comme par exemple l'ornithine qui est générée dans le processus de production de l'urée par l'arginase à partir de L-arginine. Dans l'invention, les acides aminés utilisés peuvent donc être naturels ou non. Notamment, les acides aminés naturels sont généralement de configuration L, mais au sens de l'invention, un acide aminé peut aussi bien être de configuration L ou D. De plus, R n'est bien entendu pas limité aux chaînes latérales des acides aminés naturels, mais peut être librement choisi.

Dans les motifs pseudopeptidiques des composés de formule (I), Z est soit absent (i = 0), soit présent (i=1) et alors choisi parmi

- une proline, éventuellement substituée en $\gamma$, $\beta$ ou $\delta$ par des groupements hydroxyle, amine, alkyle en $C_1$-$C_{10}$, alcényle en $C_1$-$C_{10}$, alcynyle en $C_1$-$C_{10}$, aryle en $C_5$-$C_{12}$, aralkyle en $C_5$-$C_{14}$, hétéroaryle en $C_5$-$C_{12}$ (avantageusement un hétéroaryle en $C_5$), ces groupes étant eux-mêmes éventuellement substitués par 1 à 6 substituants choisis parmi un atome d'halogène, $NO_2$, OH, un alkyle en $C_1$-$C_4$, $NH_2$, CN, un trihalométhyle, un acyloxy en $C_1$-$C_4$, un dialkylamino en $C_1$-$C_4$, un groupe guanidino, un groupe thiol;
- un acide aminé, naturel ou non, N-alkylé ;
- un acide aminé dialkylé (par exemple l'acide amino isobutyrique) ;
- un acide aminé dialkylé cyclique ; ou ,
- l'acide pipécolique ou l'un de ses dérivés ;

Par « alkyle en $C_1$-$C_i$ », on entend un radical hydrocarboné saturé linéaire ou ramifié de formule $-C_jH_{2j+1}$, où $1 \leq j \leq i$. Les alkyles en $C_1$-$C_{10}$ comprennent donc les alkyles en $C_1$ (méthyle), $C_2$ (éthyle), $C_3$ (n-propyle, ou isopropyle), $C_4$ (n-butyle, isobutyle, sec-butyle ou tert-butyle), $C_5$ (ex : n-pentyle, néopentyle, isopentyle, tert-pentyle), et $C_6$ à $C_{10}$. Par «alcényle en $C_1$-$C_{10}$ » on entend un radical hydrocarboné insaturé linéaire ou ramifié comprenant de 1 à 10 atomes de carbones et comprenant au moins une double liaison C=C. Par « alcynyle en $C_1$-$C_{10}$ » on entend un radical hydrocarboné insaturé linéaire ou ramifié comprenant de 1 à 10 atomes de carbones et comprenant au moins une triple liaison C≡C. Par « aryle en $C_5$-$C_{12}$ », on entend un radical hydrocarboné monocyclique ou polycyclique aromatique de 5 à 12 atomes de carbones. Par « aralkyle en $C_5$-$C_{14}$ », on entend la combinaison d'un alkyle et d'un aryle possédant au total 5 à 14 atomes de carbone. Par « hétéroaryle en $C_5$-$C_{12}$ », on entend un groupement aryle dont au moins un atome de carbone de la chaîne hydrocarbonée comportant normalement 5 à 12 carbones est substitué par un autre atome choisi parmi N, O, ou S. Par « hétéroaryle en $C_5$ », on entend donc un groupement aryle dont au moins un des 5 atomes de carbone de la chaîne hydrocarbonée est substitué par un autre atome choisi parmi N, O, ou S. Par « acyloxy en $C_1$-$C_4$ », on entend un groupement de formule -O(O)C-(alkyle en $C_1$-$C_4$), -O(O)C-(cycloalkyle en $C_4$-$C_{12}$), -O(O)C-(aryle en $C_4$-$C_{12}$), -O(O)C-(arylalkyle en $C_4$-$C_{12}$), ou -O(O)C-(hétéroaryle en $C_4$-$C_{12}$). Avantageusement, dans un composé de formule (I), un tel « acyloxy en $C_1$-$C_4$ » est choisi parmi un groupement de formule -O(O)C-(alkyle en $C_1$-$C_4$), -O(O)C-(cycloalkyle en $C_4$), -O(O)C-(aryle en $C_4$), -O(O)C-(arylalkyle en $C_4$), ou -O(O)C-(hétéroaryle en $C_4$). Par « dialkylamino en $C_1$-$C_4$ » on entend un radical de formule - N(alkyle en $C_1$-$C_4$)$_2$ où chaque alkyle est identique ou différent.

Par « acide aminé N-alkylé », on entend un acide aminé quelconque dont un des atomes d'hydrogène du groupement amine est substitué par une chaîne alkyle $C_1$-$C_{10}$ ou un groupement aralkyle en $C_5$-$C_{14}$, de préférence en $C_5$-$C_{10}$, notamment en $C_{10}$, éventuellement substitués. Des exemples d'acides aminés N-alkylés comprennent l'acide N-méthylglycine ou sarcosine, l'acide N-méthylisoleucine, l'acide N-méthylvaline etc... Par « acide aminé dialkylé », on entend tout acide aminé dont deux des atomes d'hydrogène (sur le carbone central ou sur le groupement amine) sont substitués par une chaîne alkyle en $C_1$-$C_{10}$ ou un groupement aralkyle en $C_5$-$C_{14}$, de préférence en $C_5$-$C_{10}$, notamment en $C_{10}$, éventuellement substitués. Des exemples d'acides aminés dialkylés comprennent l'acide 2-amino-isobutyrique (Aib), l'acide aminocyclopropane carboxylique etc...

Avantageusement, Z est présent et donc i vaut 1. Avantageusement également, quand Z est présent (i=1), alors Z est une proline, éventuellement substituée en $\gamma$, $\beta$ ou $\delta$ comme décrit précédemment.

Dans les motifs pseudopeptidiques des composés de formule (I), $Y_1$ et $Y_2$ sont choisis parmi les acides aminés à chaîne latérale basique. Par « acide aminé à chaîne latérale basique » on entend tout acide aminé naturel ou non, dont la chaîne latérale R possède une valeur de pKa supérieure à 7 (pKa(R)>7). Ainsi, tout acide aminé peut être utilisé pour $Y_1$ et $Y_2$, sous réserve que sa chaîne latérale possède une valeur de pKa supérieure à 7, de préférence supérieure à 7,5 ; supérieure à 8 ; supérieure à 8,5 ; ou supérieure à 9. Notamment, parmi les acides aminés naturels, ceux dont la chaîne latérale possède une valeur de pKa supérieure à 7 comprennent la lysine (K, pKa(R) ≈ 10,5) l'arginine (R, pKa(R) ≈ 12,5), et l'ornithine (homologue inférieur de la lysine, pKa(R) ≈ 10,8), considérés généralement comme les acides aminés naturels basiques. Ainsi, dans un mode de réalisation avantageux, $Y_1$ et $Y_2$ sont indépendamment choisis parmi l'arginine (R), la lysine (K) et l'ornithine. Encore avantageusement, $Y_1$ est une lysine (K) et $Y_2$ est une arginine (R). Mais d'autres acides aminés non naturels peuvent leur être substitués, du moment que la valeur de pKa de leur chaîne latérale R soit supérieure à 7, de préférence supérieure à 7,5 ; supérieure à 8 ; supérieure à 8,5 ; ou supérieure à 9.

Dans les composés selon l'invention, le motif pseudopeptidique essentiel à la liaison au domaine RGG de la nucléoline est le sous-motif de formule (II)

$$Y_1 \overset{\Psi}{\text{———}} (Z)i — Y_2 \quad (II)$$ ,

où $Y_1$ et $Y_2$ sont tels que définis précédemment. Cependant, la présence à l'une ou l'autre extrémité de ce sous-motif essentiel de quelques acides aminés quelconques, tels que définis précédemment, n'est pas de nature à empêcher la liaison à la nucléoline. C'est pourquoi, le sous-motif essentiel de formule (II) peut comprendre à l'une et/ou l'autre de ses extrémités de 0 à 3 acides aminés quelconques, représentés dans la formule (I) par (X)n et (X)m respectivement, où n vaut 0 ou 1 et m sont est un entier de 0 à 3. Avantageusement, le nombre d'acides aminés quelconques présents à l'une et/ou l'autre des extrémités du sous-motif essentiel de formule (II) est faible, c'est-à-dire que n vaut avantageusement 0 et m est un entier avantageusement entre 0 et 2, avantageusement 0 ou 1, avantageusement 0. Ainsi, dans un mode de réalisation avantageux, n et m sont égaux à 0.

Dans les composés selon l'invention, le sous-motif de formule (II) comprend une liaison peptidique Ψ modifiée, significativement plus résistante à au moins une protéase qu'une liaison peptidique normale.

Par « liaison peptidique normale », on entend une liaison amide de formule (-CONH-) , qui est normalement présente entre deux acides aminés au sein d'une protéine naturelle. Une telle liaison est sensible à l'action des protéases. Par « liaison peptidique Ψ modifiée », on entend une liaison chimique entre deux acides aminés de formule chimique distincte de la liaison peptidique normale de formule (-CONH-). Cette liaison Ψ modifiée est telle qu'elle est significativement plus résistante à au moins une protéase qu'une liaison peptidique normale de formule (-CONH-). Par « protéase », encore appelée « peptidase » ou « enzyme protéolytique », on entend toute enzyme qui clive les liaisons peptidiques normales des protéines. Ce processus est appelé clivage protéolytique. Cela implique l'utilisation d'une molécule d'eau ce qui classe les protéases parmi les hydrolases. Parmi les protéases, on distingue notamment les protéases appelées N-peptidases qui effectuent le clivage à l'extrémité N-terminale des protéines. Ces protéases sont particulièrement gênantes pour la stabilité in vivo de peptides sans liaisons peptidiques modifiées. C'est la raison pour laquelle les motifs pseudopeptidiques des composés de formule (I) comprennent une liaison Ψ modifiée entre $Y_1$ et Z (si i=1) ou $Y_1$ et $Y_2$ (si i=0) de façon à augmenter significativement la résistance du sous-motif de formule (II), qui est essentiel à la liaison à la nucléoline, notamment à ces N-peptidases. La liaison Ψ devrait donc permettre d'augmenter significativement la résistance à au moins une N-peptidase. Cela permet d'augmenter significativement la demi-vie des composés de formule (I) in vivo et in vitro. Notamment, le composé HB19, qui présente cette liaison Ψ modifiée, possède dans du sérum humain ou dans du sérum de veau fétal à 37°C une demi-vie supérieure à 24 heures, tandis que le même composé avec une liaison peptidique normale à la place de la liaison Ψ ne possède dans les même conditions qu'une demi-vie d'une heure.

De plus, les inventeurs on trouvé que la présence de cette liaison Ψ modifiée permet également d'augmenter significativement l'efficacité de liaison à la nucléoline. Ce phénomène pourrait être dû au fait que cela permet au compose HB19 de former un complexe irréversible avec la nucléoline.

Différentes liaisons chimiques susceptibles d'augmenter significativement la résistance à au moins une protéase sont connues. Ainsi, dans un mode de réalisation avantageux, Ψ représente une liaison réduite ($-CH_2NH-$) (ou bien ($-CH_2N-$) dans le cas où la liaison se fait au niveau d'un groupement amine secondaire comme dans le cas d'une liaison avec une proline), une liaison retro inverso (-NHCO-) une liaison méthylène oxy ($-CH_2-O-$) , une liaison thiométhylène ($-CH_2-S-$), une liaison carba ($-CH_2CH_2-$) , une liaison cétométhylène ($-CO-CH_2-$), une liaison hydroxyéthylène ($-CHOH-CH_2-$), une liaison (-N-N-) une liaison E-alcène ou une liaison (-CH=CH-). Notamment, les inventeurs ont montré que l'utilisation d'une liaison réduite ($-CH_2NH-$) permet d'augmenter significativement la résistance à au moins une protéase. Avantageusement, Ψ représente donc une liaison réduite ($-CH_2NH-$)

Bien que seule la liaison Ψ entre $Y_1$ et Z (si i=1) ou $Y_1$ et $Y_2$ (si i=0) soit systématiquement présente dans les composés de formule (I), il est également possible que d'autres liaisons peptidiques des motifs pseudopeptidiques soient modifiées tel que décrit précédemment. En particulier, au sens de l'invention, les liaisons entre acides aminés pour lesquelles rien n'est précisé peuvent être indifféremment des liaisons peptidiques normales ou des liaisons Ψ modifiées tel que décrit précédemment. La présence de liaisons Ψ additionnelles peut permettre d'augmenter encore la résistance aux protéases des composés de formules (I). Cependant, l'augmentation liée à la présence de la première liaison Y entre $Y_1$ et Z (si i=1) ou $Y_1$ et $Y_2$ (si i=0) est déjà très significative, et l'ajout d'autres liaisons Ψ complique la synthèse des motifs pseudopeptidiques et donc des composés de formule (I). La présence de liaisons Ψ additionnelles est donc possible mais facultative.

Des exemples de composés utilisables dans l'invention incluent notamment les composés (voir Figure 2 et exemples 1, 2, 3 et 4) :

- HB19 (Figure 2A, SEQ ID NO : 5, composé possédant pour support un peptide linéaire de séquence SEQ ID NO:4 sur lequel 5 motifs pseudopeptidiques KΨPR (avec Ψ = $CH_2$-N) sont liés covalamment sur le ε amino groupe de chacun des 5 résidus lysine),
- Nucant 01 (Figure 2B, composé possédant pour support un hexapeptide cyclique constitué en alternance de résidus alanine (A) de configuration D et de résidus lysine (K) de configuration L, sur lequel trois motifs pseudopeptidiques KΨPR (avec Ψ = $CH_2$-N) sont liés covalamment sur le ε amino groupe de chacun des 3 résidus lysine (K) ; voir Figure 2B),
- Nucant 2 (Figure 2C, SEQ ID NO: 20, composé possédant pour support un peptide linéaire adoptant une structure hélicoïdale de séquence SEQ ID NO :18 sur lequel 5 motifs pseudopeptidiques KΨPR (avec Ψ = $CH_2$-N) sont liés covalamment sur le ε amino groupe de chacun des 5 résidus lysine),
- Nucant 3 (Figure 2D, SEQ ID NO:21, composé possédant pour support un peptide linéaire adoptant une structure hélicoïdale de séquence SEQ ID NO:19 sur lequel 5 motifs pseudopeptidiques KΨPR (avec Ψ = $CH_2$-N) sont liés covalamment sur le ε amino groupe de chacun des 5 résidus lysine),
- Nucant 6 (Figure 2E, SEQ ID NO : 16, composé possédant pour support un peptide linéaire adoptant une structure hélicoïdale de séquence SEQ ID NO:15 sur lequel 6 motifs pseudopeptidiques KΨPR (avec Ψ = $CH_2$-N) sont liés covalamment sur le ε amino groupe de chacun des 6 résidus lysine), et
- Nucant 7 (Figure 2F, SEQ ID NO : 17, composé possédant pour support un peptide linéaire de séquence SEQ ID NO:13 sur lequel 6 motifs pseudopeptidiques KΨPR (avec Ψ = $CH_2$-N) sont liés covalamment sur le ε amino groupe de chacun des 6 résidus lysine).

Par « maladie inflammatoire », on entend toute maladie dans laquelle une réaction inflammatoire a des conséquences pathologiques pour l'organisme. Notamment, les maladies inflammatoires au sens de l'invention incluent les maladies autoimmunes (telle que le lupus ou la polyarthrite rhumatoïde), la septicémie, le choc septique, les maladies inflammatoires cardiaques (cardites, et notamment endocardite, péricardite, myocardite, en particulier les endocardites d'origine infectieuse, telles que celles induites par Staphylococcus aureus), le rejet de greffe, les traumatismes, les maladies inflammatoires articulaires (notamment les différents types d'arthrite), les maladies inflammatoires du système gastro-intestinal (notamment les colites, entérites, gastrites, gastro-entérites, et les maladies inflammatoires chroniques de l'intestin (MICI) telles que la maladie de Crohn et la recto-colite hémorragique (RCH)), les maladies inflammatoires de la peau (eczéma, dermatite de contact allergique, psoriasis, dermatose), les maladies inflammatoires des voies respiratoires (asthme, bronchite chronique, en particulier la bronchite chronique obstructive (COPD)), et les allergies.

Dans un mode de réalisation avantageux, la maladie inflammatoire est une maladie autoimmune, en particulier le lupus ou la polyarthrite rhumatoïde. Dans un autre mode de réalisation avantageux, la maladie inflammatoire est le choc septique. Dans un autre mode de réalisation avantageux, la maladie inflammatoire est une endocardite, en particulier une endocardite d'origine infectieuse, notamment du type de celles induites par Staphylococcus aureus.

Les avantages de la présente invention sont illustrés dans les figures et les exemples ci-après.

**DESCRIPTION DES FIGURES**

**Figure 1. A.** Structure de la protéine nucléoline. La nucléoline humaine est constituées de 707 acides-aminés. On peut décomposer la nucléoline en 2 domaines majeurs (3,4) : N-terminal (aa 1-308) et C-terminal (309-706). Le domaine N-terminal comporte 4 longs domaines acides, constitués d'une répétition ininterrompue d'acides glutamiques et d'acides aspartiques (A1, A2, A3, A4). Le domaine C-terminale constitué d'une alternance de régions hydrophobes et hydrophiles formant 4 domaines de liaison à l'ARN nommés RBDs (pour « RNA Binding Domains » : I, II, III, IV) et à son extremité (aa 644-707) se trouve le domaine RGG fortement basique composé de répétitions Arg-Gly-Gly. **B.** identification du domaine de liaison du composé HB19 à la nucléoline : le domaine RGG. Des constructions de la nucléoline correspondant aux parties N- et C- terminales ont été réalisées par la transcription/traduction in vitro dans le système employant les lysats des réticulocytes du lapin. Ainsi, la nucléoline complète et les parties N- et C- terminales contenant les acides aminés 1-707, 1-308 et 309-707 respectivement marqués à la [$^{35}$S] Met/Cys ont été produits. Les produits bruts marqués ont ensuite été incubés avec HB19-biotinylé, et les complexes purifiés sur une colonne d'avidine-agarose. Comme attendu, la nucléoline complète interagit avec HB19. En revanche, la partie N-terminale de la nucléoline riche en résidus acides n'interagit pas du tout avec le composé, alors que la partie C-terminale interagit avec HB19 (14). Ayant identifié que la partie C-terminale de la nucléoline contient la cible d'HB19, différentes constructions (N° 1 à 9) de cette région de la nucléoline ont été réalisées. La première construction correspond à l'ADNc codant pour la partie C-terminale de la nucléoline humaine, comprenant les 4 RBDs et le domaine RGG, en fusion avec la protéine GST (Glutathione S-Transferase) pour permettre la détection avec des anticorps dirigés contre la GST. Les autres constructions, également en fusion avec la GST, correspondent

à cette même partie mais tronquée d'un ou plusieurs domaines. Toutes ces protéines sont générées chez E.coli. La capacité d'HB19 d'interagir avec chaque construction a été testée en incubant les extraits bruts des bactéries, exprimant les différentes constructions de la nucléoline, avec HB19-biotinylé qui ensuite a été purifié par fixation sur Avidine-agarose. Ces échantillons ont ensuite été analysés par gel de polyacrylamide et la GST a été révélée par immunodétection (Western Blot) en utilisant des anticorps anti-GST. Les résultats démontrent que la présence du domaine RGG est nécessaire pour l'interaction avec HB19 avec la partie C-terminale de la nucléoline. De plus, le domaine RGG seul est suffisant pour cette interaction.

**Figure 2. A**. Structure du composé HB-19. **B**. Structure du composé trivalent Nucant 01 ayant pour support un hexapeptide cyclique constitué en alternance de résidus alanine (A) de configuration D et de résidus lysine (K) de configuration L. Trois motifs pseudopeptidiques KΨPR (avec $\Psi$ = $CH_2$-N) sont liés covalamment sur le ε amino groupe de chacun des 3 résidus lysine. **C**. Structure du composé pentavalent Nucant 2 (SEQ ID NO:10) ayant pour support un peptide linéaire adoptant une structure hélicoïdale de séquence SEQ ID NO :8 sur lequel 5 motifs pseudopeptidiques KΨPR (avec $\Psi$ = $CH_2$-N) sont liés covalamment sur le ε amino groupe de chacun des 5 résidus lysine, Ac représente un groupement $CH_3$-CO-. **D**. Structure du composé pentavalent Nucant 3 (SEQ ID NO : :11) ayant pour support un peptide linéaire adoptant une structure hélicoïdale de séquence SEQ ID NO:9 sur lequel 5 motifs pseudopeptidiques KΨPR (avec $\Psi$ = $CH_2$-N) sont liés covalamment sur le ε amino groupe de chacun des 5 résidus lysine, Ac représente un groupement $CH_3$-CO-. **E**. Structure du composé hexavalent Nucant 6 (SEQ ID NO:16) ayant pour support un peptide linéaire adoptant une structure hélicoïdale de séquence SEQ ID NO:15 sur lequel 6 motifs pseudopeptidiques KΨPR (avec $\Psi$ = $CH_2$-N) sont liés covalamment sur le ε amino groupe de chacun des 6 résidus lysine, Ac représente un groupement $CH_3$-CO-. **F.** Structure du composé hexavalent Nucant 7 (SEQ ID NO:17) ayant pour support un peptide linéaire de séquence SEQ ID NO:13 sur lequel 6 motifs pseudopeptidiques KΨPR (avec $\Psi$ = $CH_2$-N) sont liés covalamment sur le ε amino groupe de chacun des 6 résidus lysine, Ac représente un groupement $CH_3$-CO-.

**Figure 3.** Nucant 6 et Nucant 7 présentent une activité anti-nucléoline de surface supérieure à HB-19. $ID_{50}$: la concentration en μM qui inhibe 50% la nucléoline de surface. $ID_{95}$: la concentration en μM qui inhibe 95% la nucléoline de surface.

**Figure 4.** Effet inhibiteur d'HB-19, de Nucant 3, de Nucant 6 et de Nucant 7 sur l'expression cellulaire de la nucléoline par des cellules de MDA-MB 231. Des cellules MDA-MB 231 ont été cultivées dans des flacons de 75 $cm^2$ dans du DMEM contenant du sérum de veau foetal à 10%. Après 2 jours de culture, les cellules subconfluentes (environ 3 x $10^6$ cellules par flacon) ont été traitées avec 10 μM d'HB-19 (piste 1), de Nucant 3 (piste 2), de Nucant 6 (piste 3) ou de Nucant 7 (piste 4) pendant 24 ou 48 heures. Les pistes C, représentent les cellules non traitées. Après 24 ou 48 heures de traitement, les cellules ont été lavées par du PBS et incubées avec 10 ml de DMEM contenant 1% de sérum de veau foetal et HB-19 biotinylé (5 μM) pendant 45 minutes à la température ambiante. Après un lavage intensif du tapis cellulaire avec du PBS contenant 1mM EDTA (PBS-EDTA), des extraits cytoplasmiques ont été préparés à l'aide d'un tampon de lyse contenant 20 mM Tris HCl, pH 7.6, 150 mM de NaCl, 5 mM de $MgCl_2$, 0.2 mM de fluorure de phenylmethylsulfonyl, 5 mM de β-mercaptoéthanol, aprotinin (1000 U/ml) et 0.5% Triton X-100. Le complexe formé entre la nucléoline de surface et HB-19 biotinylé a été isolé par purification des extraits en utilisant de l'avidine-agarose (100 μl; ImmunoPure Immobilized Avidin, Pierce Chemical Company, USA) dans PBS-EDTA. Après 2 h d'incubation à 4°C, les échantillons d'avidine-agarose ont été lavés intensivement avec PBS-EDTA. Ces échantillons contenant la nucléoline de surface purifiée (A; matériel correspondant à 2 x $10^6$ cellules) et des extraits bruts de cellules (B et C; matériel correspondant à 4 x $10^5$ cellules) ont été dénaturés par chauffage dans le tampon d'électrophorèse contenant du SDS et analysés par SDS-PAGE. La présence de nucléoline de surface a été révélée par immunoblotting en utilisant un anticorps monoclonal D3 (A et B). L'analyse électrophorétique après coloration au bleu de Coomassie est montrée en C. La piste M correspond aux marqueurs de poids moléculaire.

**Figure 5.** Inhibition par HB-19 de la production de TNF-α par des cellules mononucléées du sang périphérique (PBMC) humaines primaires stimulées par différentes préparations de LPS. Des PBMC ont été isolés par centrifugation sur gradient de densité Ficoll à partir de sang total humain EDTA-potassium, et re-suspendus dans du milieu RPMI 1640 contenant 1% de sérum humain AB (Invitrogen). Des cellules à une concentration de $10^6$ cellules/0,5 ml, en absence (0) ou en présence (1 et 5 μM) de HB-19, ont été stimulées avec 100 ng/ml de LPS d'Escherichia coli de type 0111 :B4 et 055 :B5, et de LPS Salmonella enterica de sérotype Re 595. Les mêmes PBMC ont été stimulés avec de la PMA :Ionomycin (Phorbol 12-myristate 13-acetate :Ionomycin) à 20 ng/ml :1 μM. Les cultures de PBMC ont été incubées à 37 °C dans un incubateur à 5% de $CO_2$ et le niveau de protéine TNF-α a été mesuré par ELISA dans des surnageants de culture récoltés après 20 heures d'incubation.

**Figure 6.** Inhibition par HB-19 de la production de TNF-α et d'IL-6 par des macrophages murins du péritoine primaires stimulés par du LPS. Des macrophages murins du péritoine, en l'absence (-) ou en présence (+) de 4 μM de HB-19 ont été soit non stimulés (B4 0) ou stimulés avec du LPS d'Escherichia coli de sérotype 0111:B4 à 100 ng/ml (B4 100) et 1000 ng/ml (B4 1000). Les cultures cellulaires ont été incubées à 37 °C dans un incubateur à 5% de $CO_2$ pendant 20 heures et les niveaux de TNF-α (**A**) et d'IL-6 (**B**) ont été mesurés par ELISA.

**Figure 7.** Inhibition par Nucant 7 de la production de TNF-α et d'IL-6 par des macrophages du péritoine murin primaires stimulés par du LPS. Des macrophages du péritoine murin, en l'absence (-) ou en présence (+) de 10 μM de Nucant 7 ont été soit non stimulés (-) ou stimulés (+) avec du LPS *d'Escherichia Coli* de sérotype 0111 :B4 à 10 ng/ml, 100 ng/ml et 1000 ng/ml. Les cultures cellulaires ont été incubées à 37 °C dans un incubateur à 5% de CO2 pendant 20 heures, et les niveaux de protéine TNF-α (**A**) et d'IL-6 (**B**) ont été mesurés par ELISA.

**Figure 8.** Inhibition par HB-19 de la production d'IL-8 et de l'expression d'ICAM-1 par des cellules endothéliales vasculaires ombilicales humaines (HUVEC) stimulées par du LPS. Des cellules HUVEC, à 10 000 cellules/$cm^2$, ont été mises en culture dans des plaques 96-puit dans du milieu EBM-2 contenant 2% de sérum de veau foetal. Les cellules en absence ou en présence de 5 μM de HB-19, ont été stimulées par du LPS d'Escherichia coli de sérotype 055 :B5 à 100 ng/ml. Les cultures cellulaires ont été incubées à 37 °C dans un incubateur à 5% de $CO^2$ pendant 20 heures et les niveaux de protéine IL-8 et ICAM-1 ont été mesurés par ELISA. Des cellules HUVEC en absence ou en présence de 5 μm de HB-19 ont été utilisées comme contrôle pour montrer les niveaux de base.

**Figure 9.** Inhibition par HB-19 de la production de TNF-α et d'IL-6 par des cellules mononucléées du sang périphérique (PBMC) humaines primaires stimulées par des des bactéries Staphylococcus aureus inactivées par la chaleur (HKSA, pour « heat-killed Staphylococcus aureus »)._Des PBMC ont été isolés par centrifugation sur gradient de densité Ficoll à partir de sang total humain EDTA-potassium, et re-suspendus dans du milieu RPMI 1640 contenant 1% de sérum humain AB (Invitrogen). Des cellules à une concentration de $10^6$ cellules/0,5 ml, en absence (Contrôle) ou en présence (10 μM) de HB-19, Nucant 3, Nucant 6, ou Nucant 7, ou de dexamethasone (Dex. 1 μg/ml)) ont été stimulées avec $10^8$ particules HKSA/ml (InvivoGen, San Diego, USA). Les cultures de PBMC ont été incubées à 37 °C dans un incubateur à 5% de $CO_2$ et le niveau de protéine TNF-α (**A**) et d'IL-6 (**B**) a été mesuré par ELISA dans des surnageants de culture récoltés après 20 heures d'incubation

## EXEMPLES

### EXEMPLE 1. Synthèse du composé trivalent Nucant 01

La structure chimique du composé trivalent Nucant 01 est présentée sur la Figure 2B. Ce composé possède pour support un hexapeptide cyclique constitué en alternance de résidus alanine (A) de configuration D et de résidus lysine (K) de configuration L. Trois motifs pseudopeptidiques KΨPR (avec Ψ = CH2-NH) sont liés covalamment sur le ε amino groupe de chacun des 3 résidus lysine (K).

La synthèse du composé Nucant 01 a été réalisée par couplage covalent du motif KΨPR sur une molécule "coeur" cyclique de symétrie C3. La synthèse de la molécule coeur a été décrite par S Fournel et coll (Fournel et al-2005). Le motif KΨPR protégé a été assemblé sur une résine de type chlorotrityl par la technique classique de synthèse en phase solide selon une chimie de type Fmoc puis clivé de la résine en condition acide faible. Le motif KΨPR protégé a ensuite été couplé sur la fonction epsilon NH2 de chaque résidu lysine (K) de la molécule coeur selon une stoechiométrie 1,1 KΨPR/1 molécule cyclique. Le couplage a été réalisé selon le procédé d'activation BOP/HoBt pendant 48H. A la fin de la réaction, les groupements protecteurs des motifs KΨPR ont été clivés en acide trifluoroacétique et le composé final précipité à l'éther. La molécule Nucant 01 finalement obtenue a été purifiée par HPLC et son intégrité caractérisée par spectrométrie de masse.

### EXEMPLE 2. Synthèse des composés pentavalents Nucant 2 et Nucant 3

Deux supports peptidiques connus pour adopter une structure hélicoïdale, sur lesquels ont été ancrés les motifs KΨPR ont été assemblés en synthèse-phase solide. Ces supports sont construits à partir de motifs répétitifs de séquence Aib-Lys-Aib-Gly pour NUCANT 2 et Lys-Aib-Gly pour NUCANT 3 enchaînés cinq fois, où Aib représente l'acide 2-amino-isobutyrique. L'assemblage a été réalisé en chimie de type Boc. Les groupements Fmoc protecteurs de la chaîne latérale des résidus Lys ont ensuite été clivés par traitement pipéridine (3 fois 5 minutes) dans la DMF. Les cinq groupements εNH2 des lys ont ensuite servi à ancrer les motifs KΨPR (avec Ψ = $CH_2$-N). Le clivage acide final a été réalisé en acide fluorhydrique. Après précipitation des peptides à l'éther, dissolution en conditions aqueuses et lyophilisation, les analogues NUCANT 2 et NUCANT 3 ont été purifiés en HPLC analysés en spectrométrie de masse et lyophilisés.

### EXEMPLE 3. Synthèse des composés hexavalents Nucant 6 et Nucant 7

Ces composés hexavalents ont été obtenus en utilisant le même procédé de synthèse que celui utilisé pour les composés pentavalents Nucant 2 et 3.

**EXEMPLE 4. Nucant 6 et Nucant 7 sont des inhibiteurs plus puissants de la nucléoline de surface que HB-19**

**4.1 <u>Nucant 6 et Nucant 7 sont des inhibiteurs plus puissants au'HB-19 et inhibent l'activité de la nucléoline de surface</u>**

L'activité de la nucléoline de surface a été testée dans des cellules HeLa P4 selon la technique que nous avons décrite auparavant (Nisole et al-2000).

Les résultats, présentés dans la Figure 3, indiquent que l'inhibition de l'activité de la nucléoline de surface par Nucant 3 est comparable à celle qu'exerce HB-19. En revanche, Nucant 6 et Nucant 7 présentent une activité anti-nucléoline de surface supérieure à HB-19. En effet, HB-19 et Nucant 3 manifestent une valeur $ID_{50}$ (Inhibitory Dose à 50%) qui est entre 0,1 - 0,2 $\mu$M, tandis que Nucant 6 et Nucant 7 présentent une valeur $ID_{50}$ qui est inférieure à 0,1 $\mu$M. De plus, Nucant 6 et Nucant 7 utilisés à 0,8 $\mu$M induisent une inhibition de l'activité de la nucléoline de surface de plus que 95%.

**4.2 <u>HB-19, Nucant 3, 6, and 7 induisent une inhibition de l'expression de la nucléoline de surface dans les cellules humaines issues d'un cancer du sein, les MDA-MB 231</u>**

La nucléoline de surface joue un rôle important dans la prolifération et l'angiogénèse tumorale. HB-19 ainsi que les molécules apparentées Nucant 3, Nucant 6 et Nucant 7 se lient spécifiquement à la nucléoline de surface bloquant ainsi la croissance et l'angiogénèse tumorale. Après liaison de ces pseudopeptides à la nucléoline de surface, le complexe [pseudopeptide-nucleolin] est rapidement internalisé selon un processus actif. Les résultats sont présentés sur la Figure 4A et montrent que le traitement des cellules avec HB-19 (piste 1), Nucant 3 (piste 2), Nucant 6 (piste 3) ou Nucant 7 (piste 4), ont comme conséquence une diminution de la présence de la nucléoline de surface en comparaison avec les cellules non traitées. Cette diminution est observée après 24 heures de traitement mais également après 48 heures de traitement où dans ce cas la nucléoline devient indétectable.

Il est intéressant de noter que l'on observe une réduction sensiblement plus importante de la nucléoline de surface lorsque les cellules sont traitées pendant 24 heures avec les pseudopeptides Nucant 6 et Nucant 7 (pistes 3 et 4) comparés aux cellules traitées avec HB-19 et Nucant 3 (pistes 1 et 2). La même observation peut être faite après 48 heures de traitement. Il est important de noter que la réduction de la nucléoline de surface n'est pas la conséquence d'une diminution de la quantité intracellulaire de la nucléoline de surface. En effet, des quantités identiques de nucléoline sont retrouvées dans les extraits cellulaires des cellules traitées, ou non, par HB-19 ou par les différents Nucant (Figure 4B). Il est a noté que d'une façon équivalente, aucune différence n'est observée concernant les profils électrophorétiques des protéines extraites de cellules traitées, ou non, par HB-19 ou les différents Nucants. Ce résultat illustre que la synthèse de protéine n'est pas affectée par HB-19 ou les différents Nucants étudiés. D'autre part, il est intéressant de noter qu'aucun effet cytotoxique d'HB-19 ou des différents Nucants étudiés n'a été observé pouvant expliquer l'effet sur la diminution de la nucléoline de surface observé.

**4.3 <u>Conclusions</u>**

L'ensemble de ces résultats montre que :

a) la nucléoline est exprimée abondamment à la surface des cellules tumorales, par exemple les cellules humaines de cancer du sein (MDA-MB231).
b) le traitement par HB-19, Nucant 3, Nucant 6 et Nucant 7 induit une diminution marquée du « pool » de la nucléoline présente à la surface des cellules.
c) les pseudopeptides Nucant 6 et Nucant 7 sont plus efficaces dans l'effet de diminution du « pool » de la nucléoline présente à la surface des cellules ainsi que dans l'inhibition de l'activité de la nucléoline de surface.

**EXEMPLE 5. Activité anti-inflammatoire des composés HB19 et Nucant 7**

**5.1 <u>Inhibition par HB-19 de la production de TNF-$\alpha$ par des PBMC humains primaires stimulés par du LPS.</u>**

<u>Méthodes</u>

Des PBMC ont été isolés par centrifugation sur gradiant de densité Ficoll à partir de sang total humain EDTA-potassium, et resuspendus dans du milieu RPMI 1640 contenant 1% de sérum humain AB (Invitrogen). Des cellules à une concentration de $10^6$ cellules/0,5 ml, en absence (0) ou en présence (1 et 5 $\mu$M) de HB-19, ont été stimulées avec 100 ng/ml de LPS d'Escherichia coli de type 0111 :B4 et 055 :B5, et de LPS Salmonella enterica de serotype Re 595.

Les mêmes PBMC ont été stimulés avec de la PMA :Ionomycin (Phorbol 12-myristate 13-acetate :Ionomycin) à 20 ng/ml :1 μM. Les cultures de PBMC ont été incubées à 37 °C dans un incubateur à 5% de $CO_2$ et le niveau de protéine TNF-α a été mesuré par ELISA dans des surnageants de culture récoltés après 20 heures d'incubation.

Résultats

Les résultats (voir Figure 5) montrent que les PBMC fraîchement isolés produisent du TNF-α, et que cette production constitutive de TNF-α n'est pas affectée par HB-19. En revanche, HB-19 inhibe de façon dose dépendante la production de TNF-α par les PBMC en réponse à une stimulation par différentes préparations de LPS d'Escherichia Coli ou de Salmonella Enterica. Cet effet est spécifique, puisque HB-19 n'a pas d'effet sur la production de TNF-α par les PBMC en réponse à une stimulation par la PMA-Ionomycine.

A 5 μM de HB-19, la production de TNF-α par des PBMC humains en réponse à la stimulation par différentes préparations de LPS est inhibée à un niveau comparable au niveau de base observé en absence de stimulation par le LPS.

**5.2 Inhibition par HB-19 et Nucant 7 de la production de TNF-α et d'IL-6 par des macrophages murins du péritoine primaires stimulés par du LPS**

Méthode

Pour obtenir des macrophages stimulés, des souris balb/c de 7 à 8 semaines ont été injectées en intra-péritonéal 4 jours avant l'expérience avec 1,5 ml de solution de thioglycolate (solution saline à 3%). Les macrophages ont été récoltés dans la cavité péritonéale par lavage du péritoine avec 5 ml de milieu RPMI contenant 1% de sérum de veau foetal. Les cellules ont ensuite été mises en plaques à une densité de $10^6$ cellules/0,5 ml dans du milieu RPMI 1640, incubées à 37 °C dans un incubateur à 5% de $CO^2$, et les cellules non adhérentes ont été enlevées 2 heures plus tard.

Les macrophages, en absence (-) ou en présence (+) de 4 μM de HB-19 ou 10 μM de Nucant 7 ont été soit non stimulés soit stimulés avec du LPS d'Echerichia Coli de sérotype 0111:B4 à 10 ng/ml, 100 ng/ml et 1000 ng/ml. Les cultures cellulaires ont été incubées à 37 °C dans un incubateur à 5% de $CO_2$ pendant 20 heures, et les niveaux de protéine TNF-α et IL-6 ont été mesurés par ELISA.

Résultats

Les résultats obtenus avec HB-19 sont représentés sur la Figure 6. A 4 μM de HB-19, la production de TNF-α et d'IL-6 par des macrophages du péritoine murin en réponse à une stimulation par du LPS est significativement inhibée. Si l'on tient compte du niveau de base observé en absence de stimulation par le LPS, le degré net d'inhibition est de 72-75 % pour le TNF-α et de 68-71 % pour l'IL-6.

Les résultats obtenus avec Nucant 7 sont représentés sur la Figure 7. A 10 μM de Nucant 7, la production de TNF-α et d'IL-6 par les macrophages du péritoine murin en réponse à une stimulation par le LPS est inhibée quasiment complètement, puisque le niveau de production de cytokine observé dans les cultures traitées par Nucant 7 en réponse à la stimulation par le LPS est comparable à celui observé en absence de LPS.

Si l'on tient compte du niveau de base observé en absence de stimulation par le LPS, le degré d'inhibition à 10 μM de Nucant 7 est de plus de 95% dans les cultures stimulées à 10, 100 et 1000 ng/ml de LPS. Le fait que le degré d'inhibition n'est pas modifié en présence d'une concentration de LPS 100 fois supérieur suggère que le mécanisme d'inhibition par Nucant 7 est principalement dû à la liaison à la nucléoline de surface. En effet, si le mécanisme d'inhibition par Nucant 7 était une conséquence de l'interaction avec le LPS, alors l'effet d'inhibition serait plus faible à 100 ng/ml de LPS qu'à 10 ng/ml de LPS.

**5.3 Inhibition par HB-19 de la production d'IL-8 et de l'expression de ICAM-1 par des cellules HUVEC stimulées par du LPS.**

Méthode

Des cellules HUVEC, à 10 000 cellules/$cm^2$, ont été mises en culture dans des plaques 96-puit dans du milieu EBM-2 contenant 2% de sérum de veau foetal. Les cellules en absence ou en présence de 5 μM de HB-19, ont été stimulées par du LPS d'Echerichia coli de sérotype 055 :B5 à 100 ng/ml. Les cultures cellulaires ont été incubées à 37 °C dans un incubateur à 5% de $CO_2$ pendant 20 heures et les niveaux de protéine IL-8 et ICAM-1 ont été mesurés par ELISA. Des cellules HUVEC en absence ou en présence de 5 μm de HB-19 ont été utilisées comme contrôle pour montrer les niveaux de base.

Résultats

Si l'on tient compte du niveau de base observé en absence de stimulation par le LPS, le degré d'inhibition de la production d'IL-8 et de ICAM-1 à 5 μM de HB-19 est d'environ 50%. Ces résultats montrent donc l'efficacité potentielle de HB-19 et des composés apparentés du type Nucant en tant qu'inhibiteur de la production d'IL-8 et de ICAM-1 (voir Figure 8).

**5.4 Inhibition par HB-19 de la production de TNF-α par des PBMC humains primaires stimulés par des bactéries Staphylococcus aureus inactivées.**

L'infection par Staphylococcus aureus a été montrée comme étant une des causes majeures dans la pathogenèse de l'endocardite (Alter et al-2002, Shun et al-2005).

Les inventeurs ont donc mesuré les niveaux de TNF-α et d'IL-6 dans des cultures de PBMC humains primaires en réponse à des bactéries Staphylococcus aureus inactivées par la chaleur (HKSA, pour « heat-killed Staphylococcus aureus »), en absence (contrôle) ou en présence de 10 μM des composés HB-19, Nucant 3, Nucant 6, ou Nucant 7. En contrôle positif (Dex.), des PBMC ont également été traités avec de la dexaméthasone, qui est un glucocorticoïde avec une activité anti-inflammatoire et immunosuppressive connue.

Méthode

Des PBMC ont été isolés par centrifugation sur gradient de densité Ficoll à partir de sang total humain EDTA-potassium, et re-suspendus dans du milieu RPMI 1640 contenant 1% de sérum humain AB (Invitrogen). Des cellules à une concentration de $10^6$ cellules/0,5 ml, en absence (Contrôle) ou en présence (10 μM) de HB-19, Nucant 3, Nucant 6, Nucant 7, et dexamethasone (1 μg/ml)) ont été stimulées avec $10^8$ particules HKSA/ml (InvivoGen, San Diego, USA). Les cultures de PBMC ont été incubées à 37 °C dans un incubateur à 5% de $CO_2$ et le niveau de protéine TNF-α et IL-6 a été mesuré par ELISA dans des surnageants de culture récoltés après 20 heures d'incubation.

Résultats

Les résultats sont présentés sur la Figure 9 et montrent que tous les pseudopeptides testés (HB-19, Nucant 3, Nucant 6, et Nucant 7) permettent d'obtenir une inhibition significative de la production par les PBMC de TNF-α et d'IL-6 en réponse à une stimulation par des bactéries Staphylococcus aureus inactivées par la chaleur. Les pseudopeptides sont aussi efficaces que le médicament anti-inflammatoire classique qu'est la dexaméthasone.

Ainsi, les pseudopeptides de formule (I), tels que HB-19, Nucant 3, Nucant 6, et Nucant 7, peuvent également être utilisés en thérapie de l'inflammation cardiaque, et en particulier dans le traitement de l'endocardite d'origine infectieuse.

**5.5 Conclusion**

Ainsi, les résultats obtenus par les inventeurs montrent que les composés HB-19 et Nucant 7 sont capables d'inhiber la production de cytokines pro-inflammatoires telles que le TNF-α, l'IL-6, ainsi que la production de la chimiokine IL-8 et de la molécule d'adhésion ICAM-1 par différents types cellulaires en réponse à une stimulation par le LPS.

En outre, ces composés permettent également d'inhiber significativement la production de cytokines pro-inflammatoires telles que le TNF-α, l'IL-6 en réponse à une stimulation par des bactéries Staphylococcus aureus, un des agents responsables de nombreuses endocardites d'origine infectieuse.

Par conséquent, ces composés, ainsi que les composés apparentés de formule (I) décrits dans la présente demande, sont capables d'inhiber la production de cytokines pro-inflammatoires et de molécules impliquées dans le recrutement des leucocytes dans les foyers d'inflammation. Ces composés peuvent donc être utilisés dans des applications anti-inflammatoires, notamment dans le traitement des différentes maladies mentionnées dans la description générale.

**BIBLIOGRAPHIE**

Hovanessian, A.G., Puvion-Dutilleul, F., Nisole, S., Svab, J., Perret, E., Deng, J. S., and Krust, B. The cell-surface-expressed nucleolin is associated with the actin cytoskeleton. (2000) Exp. Cell Res. 261, 312-328.

Nisole, S., Krust, B., Callebaut, C., Guichard, G., Muller, S., Briand, J. P., and Hovanessian, A. G. The anti-HIV

composé HB-19 forms a complex with the cell-surface-expressed nucleolin independent of heparan sulfate proteoglycans. (1999) J. Biol. Chem. 274, 27875-27884.

Ginisty, H., Sicard, H., Roger, B., and Bouvet, P. Structure and functions of nucleolin. (1999) J. Cell Science 112, 761-772.

Srivastava, M., and Pollard, H. B. Molecular dissection of nucleolin's role in growth and cell proliferation: new insights. (1999) FASEB J. 13, 1911-1922;

Nisole, S., Krust, B., and Hovanessian, A. G. Anchorage of HIV on permissive cells leads to coaggregation of viral particles with surface nucleolin at membrane raft microdomains. (2002) Exp. Cell Res. 276, 155-173.

Callebaut, C., Jacotot, E., Krust, B., Guichar, G., Blanco, J., Svab, J., Muller, S., Briand, J. P., and Hovanessian, A. G. Composé TASP inhibitors of HIV entry bind specifically to a 95-kDa cell surface protein. (1997) J. Biol. Chem. 272, 7159-7166.

Callebaut, C., Blanco, J., Benkirane, N., Krust, B., Jacotot, E., Guichard, G., Seddiki, N., Svab, J., Dam, E., Muller, S., Briand, J. P., and Hovanessian, A. G. Identification of V3 loop-binding proteins as potential receptors implicated in the binding of HIV particles to CD4(+) cells. (1998) J. Biol. Chem. 273, 21988-2199.

Said, A. E., Krust, B., Nisole, S., Briand, J. P., and Hovanessian, A. G. The anti-HIV cytokine midkine binds the cell surface-expressed nucleolin as a low affinity receptor. (2002) J. Biol. Chem. 277, 37492-37502.

Said, E. A., Courty, J., Svab, J., Delbé, J., Krust, B., and Hovanessian, A. G. Pleiotrophin inhibits HIV infection by binding the cell surface-expressed nucleolin. (2005) FEBS J. 272, 4646-4659.

Legrand, D., Vigie, K., Said, E. A., Elass, E., Masson, M., Slomianny, M. C., Carpentier, M., Briand, J. P., Mazurier, J., and Hovanessian, A. G. Surface nucleolin participates in both the binding and endocytosis of lactoferrin in target cells. (2004) Eur. J. Biochem. 271, 303-317.

Nisole, S., Krust, B., Dam, E., Blanco, A., Seddiki, N., Loaec, S., Callebaut, C., Guichard, G., Muller, S., Briand, J. P., and Hovanessian, A. G. The HB-19 composé 5[Kpsi(CH2N)PR]-TASP inhibits attachment of T lymophocyte- and macrophage-tropic HIV to permissive cells. (2000) AIDS Res. Hum. Retroviruses 16, 237-249.

Nisole, S., Said, E. A., Mische, C., Prevost, M. C., Krust, B., Bouvet, P., Bianco, A., Briand, J. P., and Hovanessian, A. G. The anti-HIV pentameric composé HB-19 binds the C-terminal end of nucleolin and prevents anchorage of virus particles in the plasma membrane of target cells. (2002) J. Biol. Chem. 277, 20877-20886;

Huang Y, Shi H, Zhou H, Song X, Yuan S, Luo Y. The angiogenesis function of nucleolin is mediated by vascular endothelial growth factor and nonmuscle myosin. (2006) Blood 107, 3564-3571.

Seko Y, Cole S, Kasprzak W, Shapiro BA, Ragheb JA. The role of cytokine mRNA stability in the pathogenesis of autoimmune disease. Autoimmun Rev 2006;5:299-305.

Rot A. Neutrophil attractant/activation protein-1 (intrleukin-8) induces in vitro neutrophil migration by haptotactic mechanism. Eur J Immunol 1993;23:303-06.

Elass E, Masson M, Mazurier J, Legrand D. Lactoferrin inhibits the lipposaccharaide-induced expression and proteoglycan-binding ability of IL-8 in human entothelial cells. Infect Immun 2002;70:1860-66.

Kevil CG, Patel RP, Bullard DC. Essential role of ICAM-1 in mediating monocyte adhesion to aortic endothelial cells. Am J Physiol Cell Physiol 2001;281:C1442-47.

Bucklin SE, Silverstein R, Morrison DC. An interleukin-6-induced acute-phase response does not confer protection against lipopolysaccharide lethality. Infect Immun 1993;61:3184-89.

Karima R, Matsumoto S, Higashi H, Matsushima K. The molecular pathogenesis of endotoxic shock and organ failure. Mol Med Today 1999;5:123-32.

Zanotti S, Kulmar A, Kumar A. Cytokine modulation in sepsis and septic shock. Expert Opin Investig Drugs 2002;11:1061-75.

Alter, P, Hoeschen, J., Ritter, M., Maisch, B. Usefulness of cytokines interleukin-6 and interleukin-2R concentrations in diagnosing active infective endocarditis involving native valves. (2002) Am. J. Cardiol. 89, 1400-1404

Shun, C.T., Lu, S.Y., Yeh, C.Y., Chaing, C.P., Chia, J.S., Chen, J.Y. Glucosyltransferases of viridans streptococci are modulins of interleukin-6 induction in infective endocarditis. (2005) Infec. Immun. 73, 3261-3270.

Kannan K, Ortmann RA, Kimpel D. Animal models of rheumatoid arthritis and their relevance to human disease. Pathophysiology 2005;12:167-81.

Fournel, S. et al. C3-symmetric peptide scaffolds are functional mimetics of trimeric CD40L. (2005) Nat Chem Biol 1, 377-382.

## Revendications

**1.** Utilisation d'un composé synthétique multivalent comprenant ou constitué d'un support sur lequel sont greffés au moins 3 motifs pseudopeptidiques, ledit composé étant de formule (I) :

$$[(X)_n—Y_1\overset{\Psi}{—}(Z)_i—Y_2\text{-}(X)_m]_k—\text{Support} \qquad (I)$$

,

où
chaque X représente indépendamment un acide aminé quelconque ;
$Y_1$ et $Y_2$ sont indépendamment choisis parmi les acides aminés à chaîne latérale basique ;
Z est choisi parmi

- une proline, éventuellement substituée en $\gamma$, $\beta$ ou $\delta$ par des groupements hydroxyle, amine, alkyle en $C_1$-$C_{10}$, alcényle en $C_1$-$C_{10}$, alcynyle en $C_1$-$C_{10}$, aryle en $C_5$-$C_{12}$, aralkyle en $C_5$-$C_{14}$, hétéroaryle en $C_5$-$C_{12}$, ces groupes étant eux-mêmes éventuellement substitués par 1 à 6 substituants choisis parmi un atome d'halogène, $NO_2$, OH, un alkyle en $C_1$-$C_4$, $NH_2$, CN, un trihalomèthyle, un acyloxy en $C_1$-$C_4$, un dialkylamino en $C_1$-$C_4$, un groupe guanidino, un groupe thiol;
- un acide aminé, naturel ou non, N-alkylé ;
- un acide aminé dialkylé ;
- un acide aminé dialkylé cyclique ; ou
- l'acide pipécolique ou l'un de ses dérivés ;

n et i sont indépendamment 0 ou 1 ;
m est un entier entre 0 et 3 ;
k est un entier supérieur ou égal à 3 ; et
$\Psi$ représente une liaison peptidique modifiée, significativement plus résistante à au moins une protéase qu'une liaison peptidique normale,
pour la fabrication d'un médicament destiné au traitement des maladies inflammatoires.

**2.** Utilisation selon la revendication 1, **caractérisée en ce que** ledit support est choisi parmi peptide linéaire ou un peptide cyclique, un peptoïde linéaire ou cyclique, un foldamère, un polymère linéaire ou un dendrimère sphérique, un sucre, ou une nanoparticule.

**3.** Utilisation selon la revendication 2, **caractérisée en ce que** ledit support est choisi parmi un peptide linéaire ou un peptide circulaire.

**4.** Utilisation selon la revendication 3, **caractérisée en ce que** ledit support est choisi parmi un hexapeptide cyclique constitué en alternance de résidus alanine (A) de configuration D et de résidus lysine (K) de configuration L, ou un peptide linéaire de séquence SEQ ID NO :1, SEQ ID NO :2, SEQ ID NO :3, SEQ ID NO :4, SEQ ID NO :8, SEQ ID NO :9, SEQ ID NO :13, SEQ ID NO :14, SEQ ID NO :15, SEQ ID NO :18, ou SEQ ID NO :19.

**5.** Utilisation selon l'une quelconque des revendications 1 à 4, **caractérisée en ce que** lesdits motifs pseudopeptidiques sont greffés directement sur ledit support.

**6.** Utilisation selon l'une quelconque des revendications 1 à 4, **caractérisée en ce que** lesdits motifs pseudopeptidiques sont greffés sur ledit support par l'intermédiaire d'un espaceur.

**7.** Utilisation selon l'une quelconque des revendications 1 à 6, **caractérisée en ce que** k est compris entre 3 et 8, avantageusement k est compris entre 5 et 6.

**8.** Utilisation selon l'une quelconque des revendications 1 à 7, **caractérisée en ce que** i vaut 1 et Z est une proline (P).

**9.** Utilisation selon l'une quelconque des revendications 1 à 8, **caractérisée en ce que** $Y_1$ et $Y_2$ sont indépendamment choisis parmi l'arginine (R) et la lysine (K), avantageusement $Y_1$ est une lysine (K) et $Y_2$ est une arginine (R).

**10.** Utilisation selon l'une quelconque des revendications 1 à 9, **caractérisée en ce que** n et m sont égaux à 0.

**11.** Utilisation selon l'une quelconque des revendications 1 à 10, **caractérisée en ce que** Ψ représente une liaison réduite ($-CH_2NH-$) , une liaison retro inverso (-NHCO-), une liaison méthylène oxy ($-CH_2-O-$) , une liaison thiométhylène ($-CH_2-S-$) , une liaison carba ($-CH_2CH_2-$) , une liaison cétométhylène ($-CO-CH_2-$), une liaison hydroxyéthylène ($-CHOH-CH_2-$), une liaison (-N-N-) une liaison E-alcène ou une liaison (-CH=CH-).

**12.** Utilisation selon la revendication 1, **caractérisée en ce que** le composé est choisi parmi les composés dont la structure est décrite sur la Figure 2A (SEQ ID NO :5), la Figure 2B, la Figure 2C (SEQ ID NO:20), la Figure 2D (SEQ ID NO :21), la Figure 2E (SEQ ID NO :16), ou la Figure 2F (SEQ ID NO :17).

**13.** Utilisation selon l'une quelconque des revendications 1 à 12, **caractérisée en ce que** ladite maladie inflammatoire est choisie parmi les maladies autoimmunes, la septicémie, le choc septique, les maladies inflammatoires cardiaques, le rejet de greffe, les traumatismes, les maladies inflammatoires articulaires, les maladies inflammatoires du système gastro-intestinal, les maladies inflammatoires de la peau, les maladies inflammatoires des voies respiratoires, et les allergies.

**14.** Utilisation selon la revendication 13, **caractérisée en ce que** ladite maladie inflammatoire est une maladie autoimmune, avantageusement ladite maladie auto-immune est le lupus ou la polyarthrite rhumatoïde.

**15.** Utilisation selon la revendication 13, **caractérisée en ce que** ladite maladie inflammatoire est le choc septique ou une endocardite.

## Patentansprüche

**1.** Verwendung einer mehrwertigen synthetischen Verbindung, die einen Träger umfasst oder von diesem gebildet ist, auf den mindestens drei Pseudopeptidmotive gepfropft sind, wobei die Verbindung der Formel (I) ist:

$$[(X)_n - Y_1 \overset{\Psi}{-} (Z)_i - Y_2 \text{-} (X)_m]_k - \text{Träger} \qquad (I),$$

wobei
jedes X unabhängig eine beliebige Aminosäure darstellt,
$Y_1$ und $Y_2$ unabhängig aus den Aminosäuren mit basischer Seitenkette ausgewählt sind,
Z ausgewählt ist aus

- einem Prolin, in γ, β oder δ eventuell durch Hydroxyl-, Amin-, $C_1$-$C_{10}$-Alkyl-, $C_1$-$C_{10}$-Alcenyl-, $C_1$-$C_{10}$-Alcynyl-, $C_5$-$C_{12}$-Aryl-, $C_5$-$C_{14}$-Aralkyl-, $C_5$-$C_{12}$-Heteroarylgruppen substituiert, wobei diese Gruppen selbst eventuell durch 1 bis 6 Substituenten substituiert sind, die aus einem Halogenatom, $NO_2$, OH, einem $C_1$-$C_4$-Alkyl, $NH_2$, CN, einem Trihalomethyl, einem $C_1$-$C_4$-Alcyloxy, einem $C_1$-$C_4$-Dialkylamin, einer Guanidinogruppe, einer Thiolgruppe ausgewählt sind,
- einer natürlichen oder nicht natürlichen N-alkylierten Aminosäure,
- einer dialkylierten Aminosäure,
- einer zyklischen dialkylierten Aminosäure, oder
- der Pipecolinsäure oder einem ihrer Derivate,

n und i unabhängig 0 oder 1 sind,
m eine Ganzzahl zwischen 0 und 3 ist,
k eine Ganzzahl größer oder gleich 3 ist, und
Ψ eine modifizierte, gegenüber mindestens einer Protease signifikant resistentere Peptidbindung als eine normale Peptidbindung darstellt,

für die Herstellung eines Arzneimittels, das für die Behandlung von entzündlichen Erkrankungen bestimmt ist.

2. Verwendung nach Anspruch 1, **dadurch gekennzeichnet, dass** der Träger aus linearem Peptid oder einem zyklischen Peptid, einem linearen oder zyklischen Peptoid, einem Foldamer, einem linearen Polymer oder einen sphärischen Dendrimer, einem Zucker oder einem Nanopartikel ausgewählt ist.

3. Verwendung nach Anspruch 2, **dadurch gekennzeichnet, dass** der Träger aus einem linearen Peptid oder einem zirkulären Peptid ausgewählt ist.

4. Verwendung nach Anspruch 3, **dadurch gekennzeichnet, dass** der Träger aus einem zyklischen Hexapeptid, das alternierend aus Alaninrückständen (A) der Konfiguration D und Lysinrückständen (K) der Konfiguration L gebildet ist, oder einem linearen Peptid der Sequenz SEQ ID NO :1, SEQ ID NO :2, SEQ ID NO :3, SEQ ID NO :4, SEQ ID NO :8, SEQ ID NO :9, SEQ ID NO :13, SEQ ID NO :14, SEQ ID NO :15, SEQ ID NO :18 oder SEQ ID NO :19 ausgewählt ist.

5. Verwendung nach einem der Ansprüche 1 bis 4, **dadurch gekennzeichnet, dass** die Pseudopeptidmotive direkt auf den Träger gepfropft sind.

6. Verwendung nach einem der Ansprüche 1 bis 4, **dadurch gekennzeichnet, dass** die Pseudopeptidmotive mittels eines Spacers auf den Träger gepfropft sind.

7. Verwendung nach einem der Ansprüche 1 bis 6, **dadurch gekennzeichnet, dass** k zwischen 3 und 8 inklusive ist, wobei k in vorteilhafter Weise zwischen 5 und 6 inklusive ist.

8. Verwendung nach einem der Ansprüche 1 bis 7, **dadurch gekennzeichnet, dass** i 1 entspricht und Z ein Prolin (P) ist.

9. Verwendung nach einem der Ansprüche 1 bis 8, **dadurch gekennzeichnet, dass** $Y_1$ und $Y_2$ unabhängig aus dem Arginin (R) und dem Lysin (K) ausgewählt sind, wobei in vorteilhafte Weise $Y_1$ ein Lysin (K) ist und $Y_2$ ein Arginin (R) ist.

10. Verwendung nach einem der Ansprüche 1 bis 9, **dadurch gekennzeichnet, dass** n und m gleich 0 sind.

11. Verwendung nach einem der Ansprüche 1 bis 10, **dadurch gekennzeichnet, dass** $\Psi$ eine reduzierte Bindung ($-CH_2NH-$), eine retroinverse Bindung (-NHCO-), eine Oxymethylenbindung ($-CH_2-O-$), eine Thiomethylenbindung ($-CH_2-S-$), eine Carbabindung ($-CH_2CH_2-$), eine Cetomethylenbindung ($-CO-CH_2-$), eine Hydroxyethylenbindung ($-CHOH-CH_2-$), eine Bindung (-N-N-), eine E-Alcen-Bindung oder eine Bindung (-CH=CH-) darstellt.

12. Verwendung nach Anspruch 1, **dadurch gekennzeichnet, dass** die Verbindung aus den Verbindungen ausgewählt ist, deren Struktur auf der Figur 2A (SEQ ID NO :5), der Figur 2B, der Figur 2C (SEQ ID NO :20), der Figur 2D (SEQ ID NO :21), der Figur 2E (SEQ ID NO :16) oder der Figur 2F (SEQ ID NO :17) beschrieben ist.

13. Verwendung nach einem der Ansprüche 1 bis 12, **dadurch gekennzeichnet, dass** die entzündliche Erkrankung aus den Autoimmunerkrankungen, der Blutvergiftung, dem septischen Schock, den entzündlichen Herzerkrankungen, der Transplantatabstoßung, den Verletzungen, den entzündlichen Gelenkerkrankungen, den entzündlichen Erkrankungen des Magen-Darm-Systems, den entzündlichen Erkrankungen der Haut, den entzündlichen Erkrankungen der Atemwege und den Allergien ausgewählt ist.

14. Verwendung nach Anspruch 13, **dadurch gekennzeichnet, dass** die entzündliche Erkrankung eine Autoimmunerkrankung ist, wobei die Autoimmunerkrankung in vorteilhafter Weise der Lupus oder die rheumatische Polyarthritis ist.

15. Verwendung nach Anspruch 13, **dadurch gekennzeichnet, dass** die entzündliche Erkrankung der septische Schock oder eine Endokarditis ist.

## Claims

1. Use of a multivalent synthetic compound comprising or consisting of a support on which at least 3 pseudopeptide units are grafted, said compound being of formula (I):

$$[(X)_n\text{—}Y_1\overset{\Psi}{\text{—}}(Z)_i\text{—}Y_2\text{-}(X)_m]_k\text{—Support} \qquad (I),$$

where
each X independently represents any amino acid;
$Y_1$ and $Y_2$ are independently selected from amino acids having a basic side chain;
Z is selected from

- proline, possibly substituted at γ, β or δ by hydroxyl, amine, $C_1$-$C_{10}$ alkyl, $C_1$-$C_{10}$ alkenyl, $C_1$-$C_{10}$ alkynyl, $C_5$-$C_{12}$ aryl, $C_5$-$C_{14}$ aralkyl, $C_5$-$C_{12}$ heteroaryl groups, these groups being themselves possibly substituted by 1 to 6 substituents selected from a halogen atom, $NO_2$, OH, $C_1$-$C_4$ alkyl, $NH_2$, CN, trihalomethyl, $C_1$-$C_4$ akyloxy, $C_1$-$C_4$ dialkylamino, guanidino group, thiol group;
- N-alkylamino acid, natural or not;
- dialkylamino acid;
- cyclic dialkylamino acid; or
- pipecolic acid or derivatives thereof;

n and i are independently 0 or 1;
m is an integer between 0 and 3;
k is an integer greater than or equal to 3; and
Ψ represents a modified peptide bond significantly more resistant to at least one protease than a standard peptide bond,
for the manufacture of a medication intended for the treatment of inflammatory diseases.

2. Use according to claim 1, **characterised in that** said support is selected from a linear peptide or a cyclic peptide, a linear or cyclic peptoid, a foldamer, a linear polymer or a spherical dendromer, a sugar or a nanoparticle.

3. Use according to claim 2, **characterised in that** said support is selected from a linear peptide or a circular peptide.

4. Use according to claim 3, **characterised in that** said support is selected from a cyclic hexapeptide consisting of alternating alanine (A) residues of configuration D and lysine (K) residues of configuration L, or a linear peptide of sequence SEQ ID NO: 1, SEQ ID NO: 2, SEQ ID NO: 3, SEQ ID NO: 4, SEQ ID NO: 8, SEQ ID NO: 9, SEQ ID NO: 13, SEQ ID NO: 14, SEQ ID NO: 15, SEQ ID NO: 18, or SEQ ID NO: 19.

5. Use according to any one of claims 1 to 4, **characterised in that** said pseudopeptide units are grafted directly on said support.

6. Use according to any one of claims 1 to 4, **characterised in that** said pseudopeptide units are grafted on said support by means of a spacer.

7. Use according to any one of claims 1 to 6, **characterised in that** k is between 3 and 8, advantageously k is between 5 and 6.

8. Use according to any one of claims 1 to 7, **characterised in that** i equals 1 and Z is proline (P).

9. Use according to any one of claims 1 to 8, **characterised in that** $Y_1$ and $Y_2$ are independently selected from arginine (R) and lysine (K), advantageously $Y_1$ is lysine (K) and $Y_2$ is arginine (R).

10. Use according to any one of claims 1 to 9, **characterised in that** n and m are equal to 0.

11. Use according to any one of claims 1 to 10, **characterised in that** Ψ represents a reduced bond ($-CH_2NH-$), a retro-inverso bond (-NHCO-), a methyleneoxy bond ($-CH_2-O-$), a thiomethylene bond ($-CH_2-S-$), a carba bond ($-CH_2CH_2-$), a ketomethylene bond ($-CO-CH_2-$), a hydroxyethylene bond ($-CHOH-CH_2-$), a (-N-N-) bond, an E-alkene bond or a (-CH=CH-) bond.

12. Use according to claim 1, **characterised in that** the compound is selected from compounds whose structure is

described in Figure 2A (SEQ ID NO: 5), Figure 2B, Figure 2C (SEQ ID NO: 20), Figure 2D (SEQ ID NO: 21), Figure 2E (SEQ ID NO: 16), or Figure 2F (SEQ ID NO: 17).

**13.** Use according to any one of claims 1 to 12, **characterised in that** said inflammatory disease is selected from autoimmune diseases, septicaemia, septic shock, cardiac inflammatory diseases, graft rejection, trauma, inflammatory diseases of the joints, inflammatory diseases of the gastrointestinal system, inflammatory diseases of the skin, inflammatory diseases of the respiratory system and allergies.

**14.** Use according to claim 13, **characterised in that** said inflammatory disease is an autoimmune disease, advantageously said autoimmune disease is lupus or rheumatoid polyarthritis.

**15.** Use according to claim 13, **characterised in that** said inflammatory disease is septic shock or endocarditis.

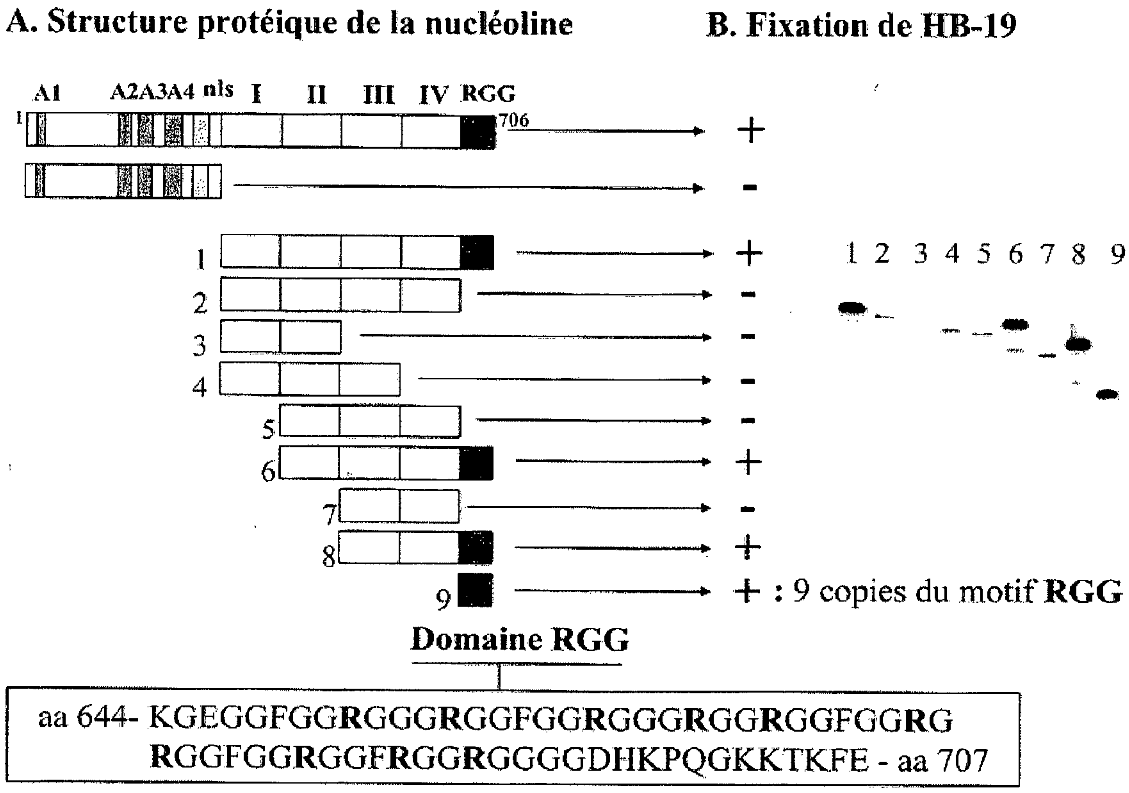


aa 644- KGEGGFGG**R**GGG**R**GGFGG**R**GGG**R**GG**R**GGFGG**R**G
**R**GGFGG**R**GGF**R**GG**R**GGGGDHKPQGKKTKFE - aa 707

**Figure 1**

A

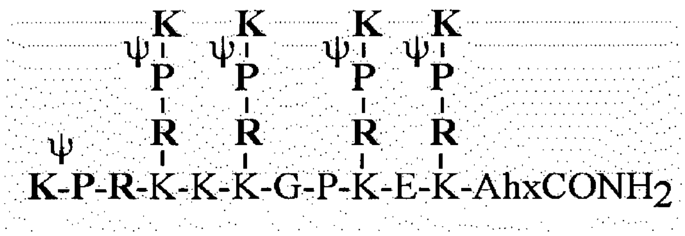

**HB-19**

B

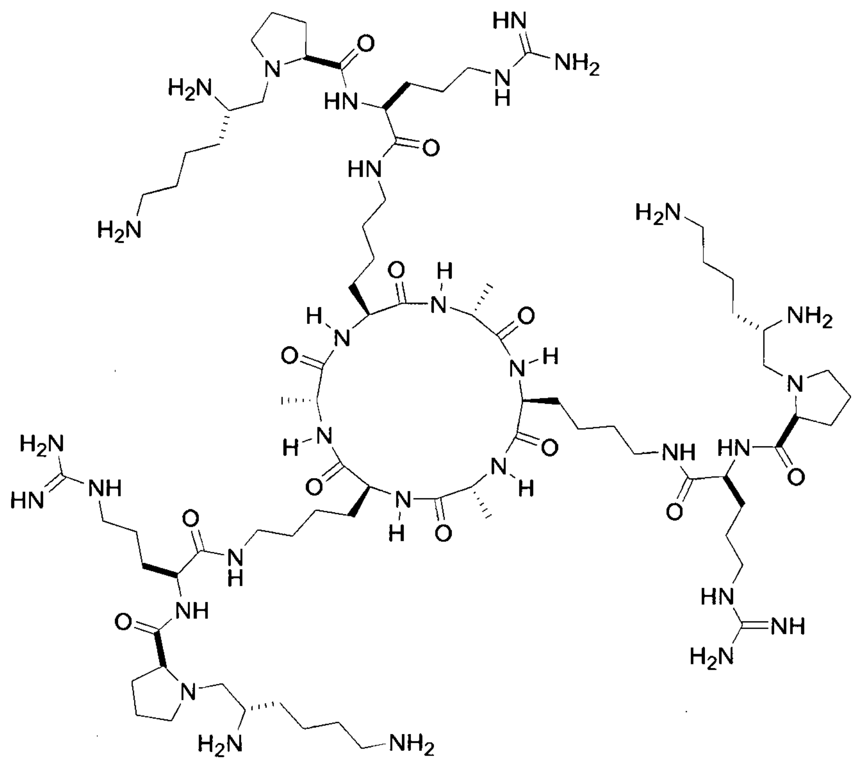

**Nucant 01**

**Figure 2**

C

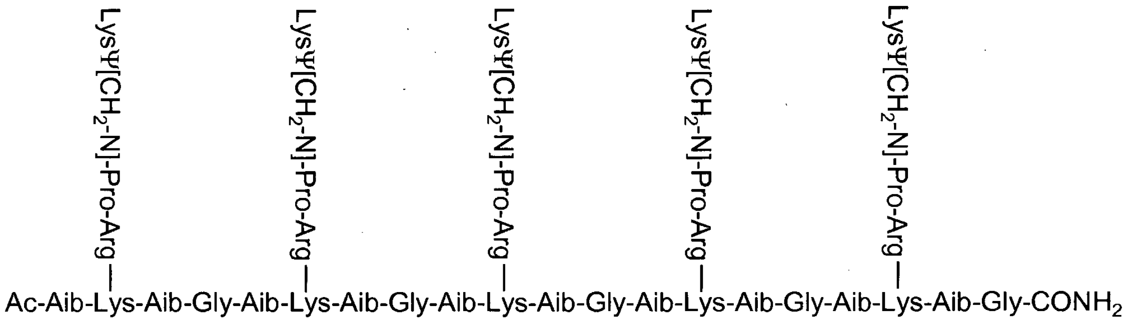

**Nucant 2**

D

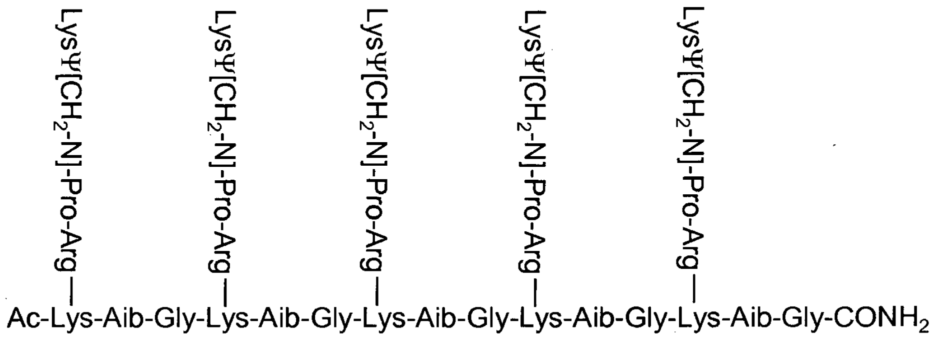

**Nucant 3**

**Figure 2 (suite)**

E

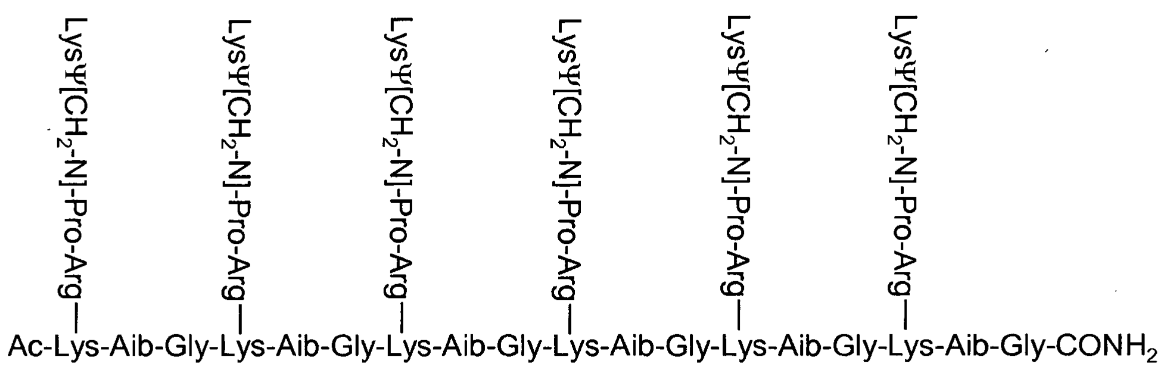

Nucant 6

F

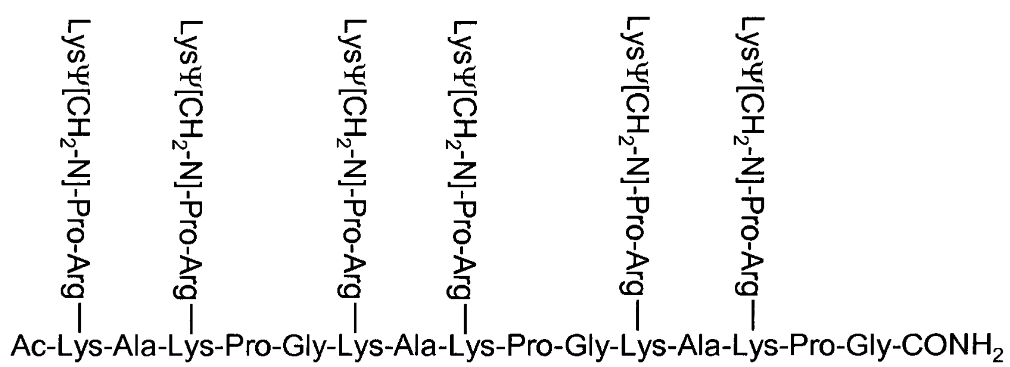

Nucant 7

Figure 2 (suite)

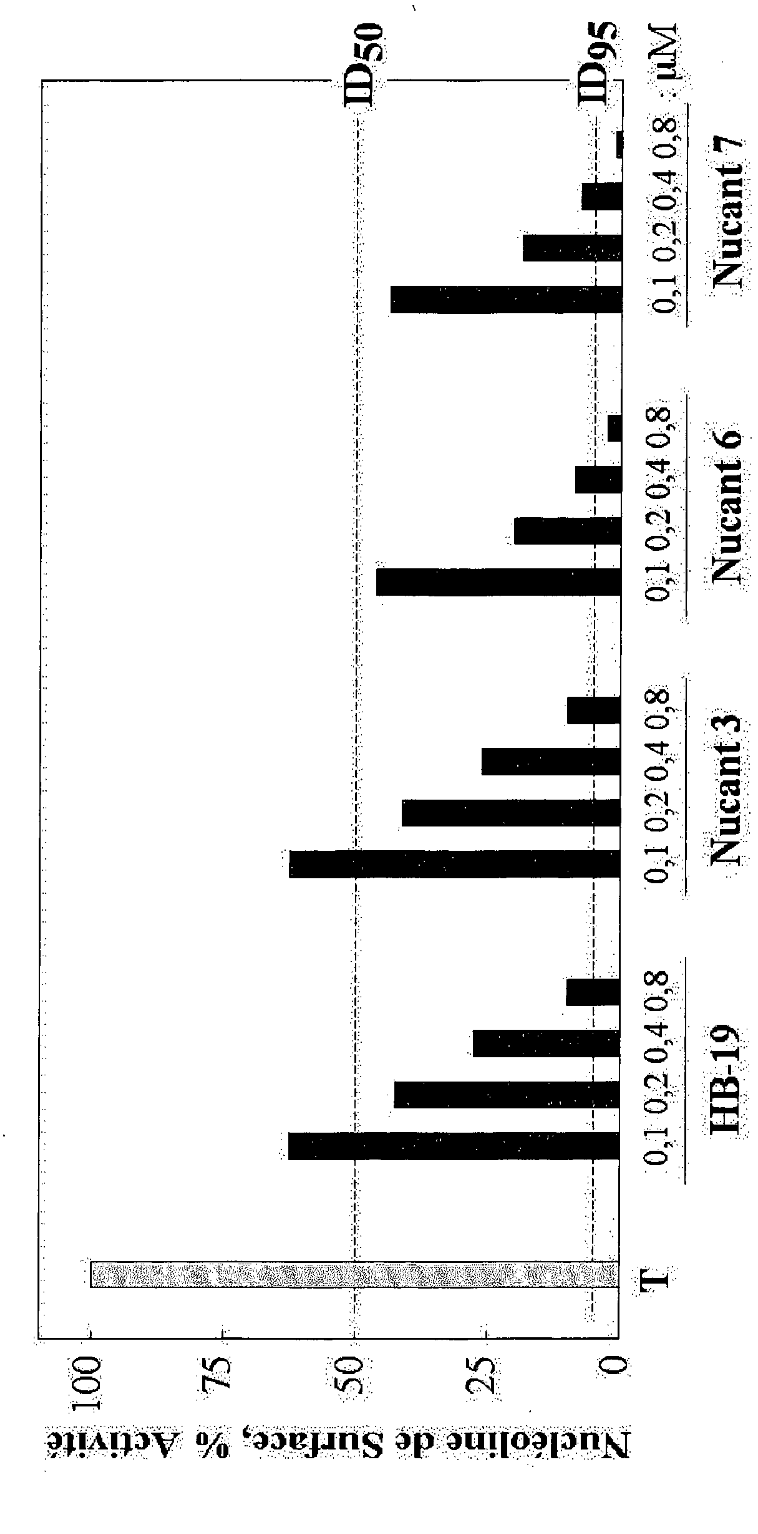
Nucléoline de Surface, % Activité
100
75
50
25
0
ID50
ID95
T
0,1 0,2 0,4 0,8
HB-19
0,1 0,2 0,4 0,8
Nucant 3
0,1 0,2 0,4 0,8
Nucant 6
0,1 0,2 0,4 0,8 : µM
Nucant 7

Figure 3

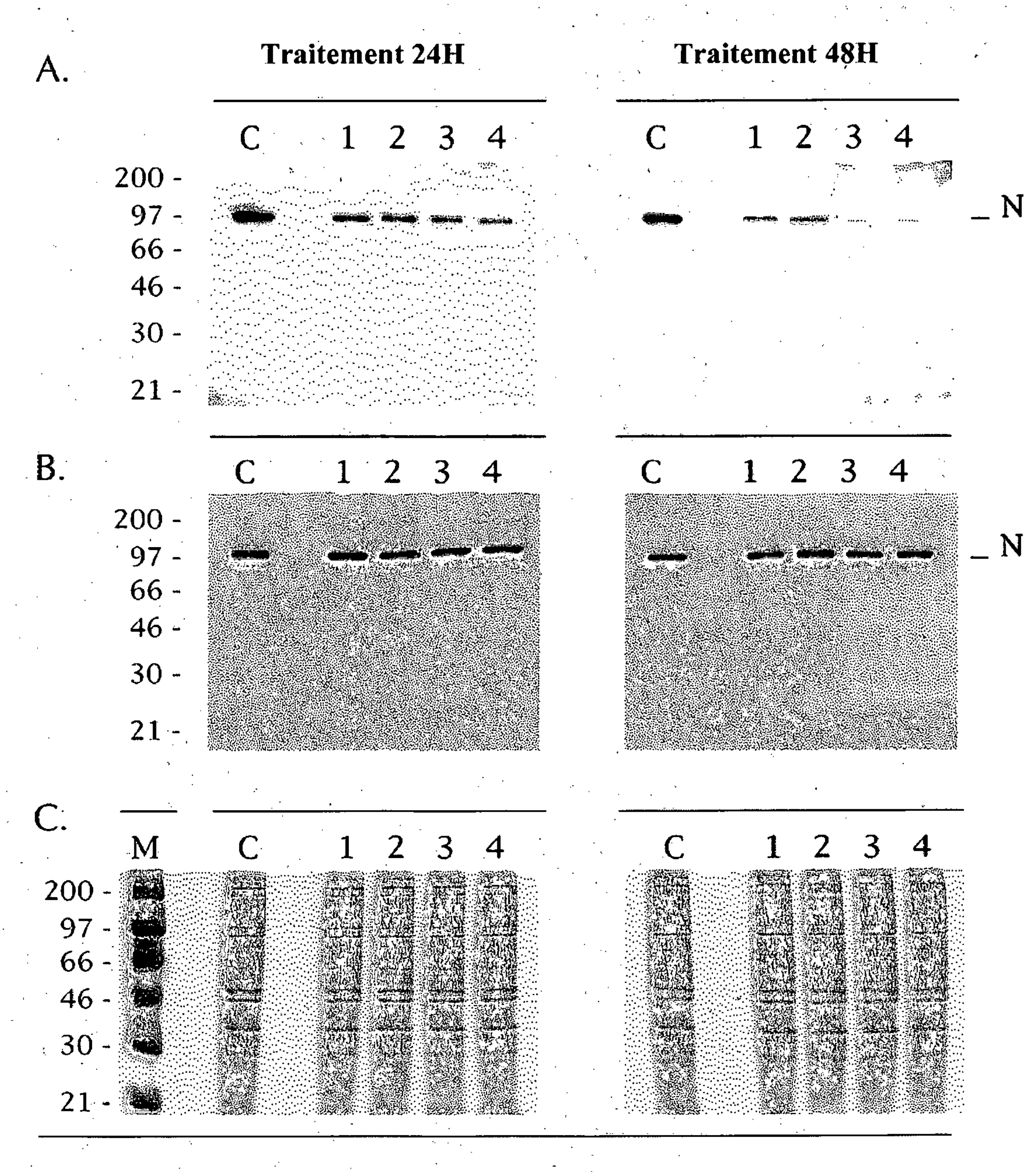
A.
Traitement 24H
Traitement 48H
C 1 2 3 4
C 1 2 3 4
200 -
97 -
66 -
46 -
30 -
21 -
N
B.
C 1 2 3 4
C 1 2 3 4
200 -
97 -
66 -
46 -
30 -
21 -
N
C.
M
C 1 2 3 4
C 1 2 3 4
200 -
97 -
66 -
46 -
30 -
21 -

**Figure 4**

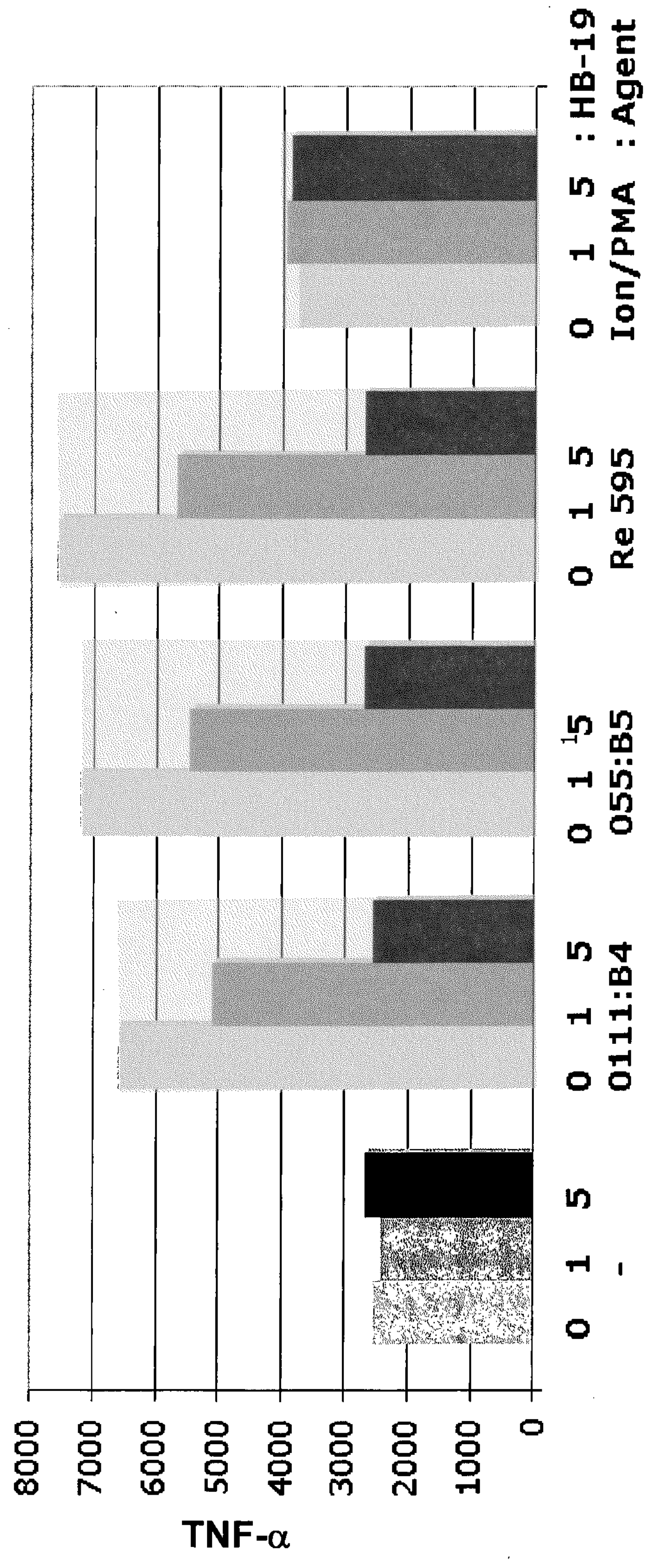
TNF-α
8000
7000
6000
5000
4000
3000
2000
1000
0
0 1 5
-
0 1 5
0111:B4
0 1 5
055:B5
0 1 5
Re 595
0 1 5 : HB-19
Ion/PMA : Agent

Figure 5

A

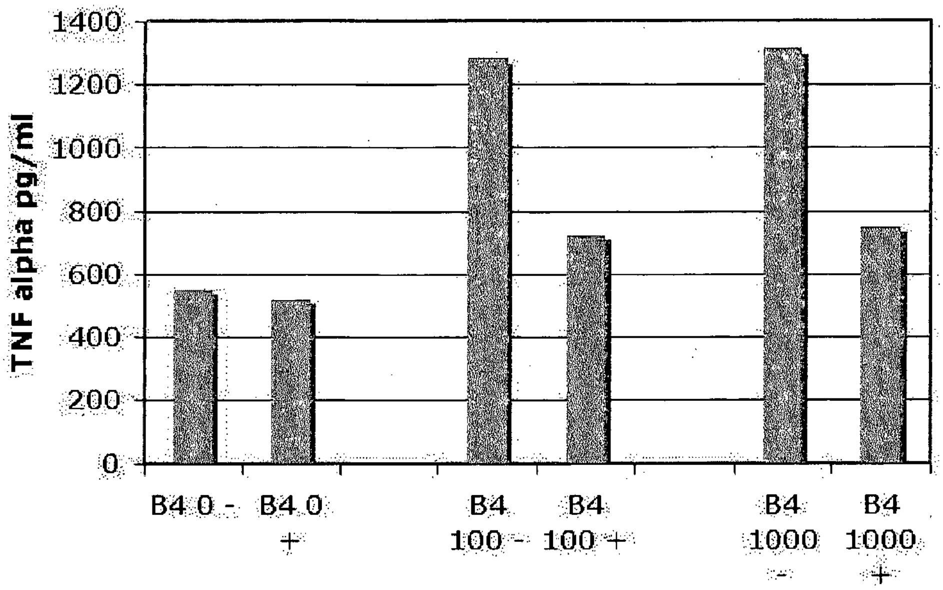
TNF alpha pg/ml
1400
1200
1000
800
600
400
200
0
B4 0 -
B4 0 +
B4 100 -
B4 100 +
B4 1000 -
B4 1000 +

B

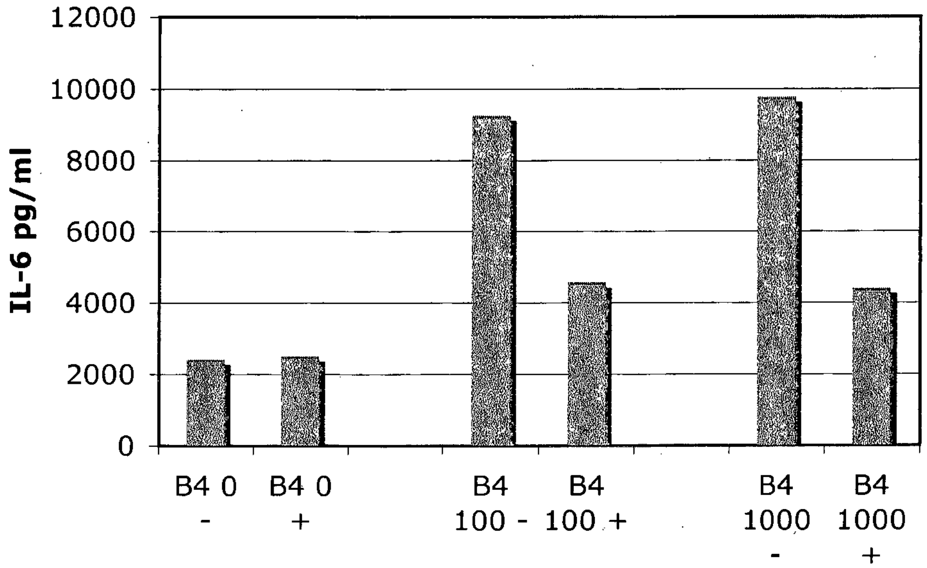
IL-6 pg/ml
12000
10000
8000
6000
4000
2000
0
B4 0 -
B4 0 +
B4 100 -
B4 100 +
B4 1000 -
B4 1000 +

**Figure 6**

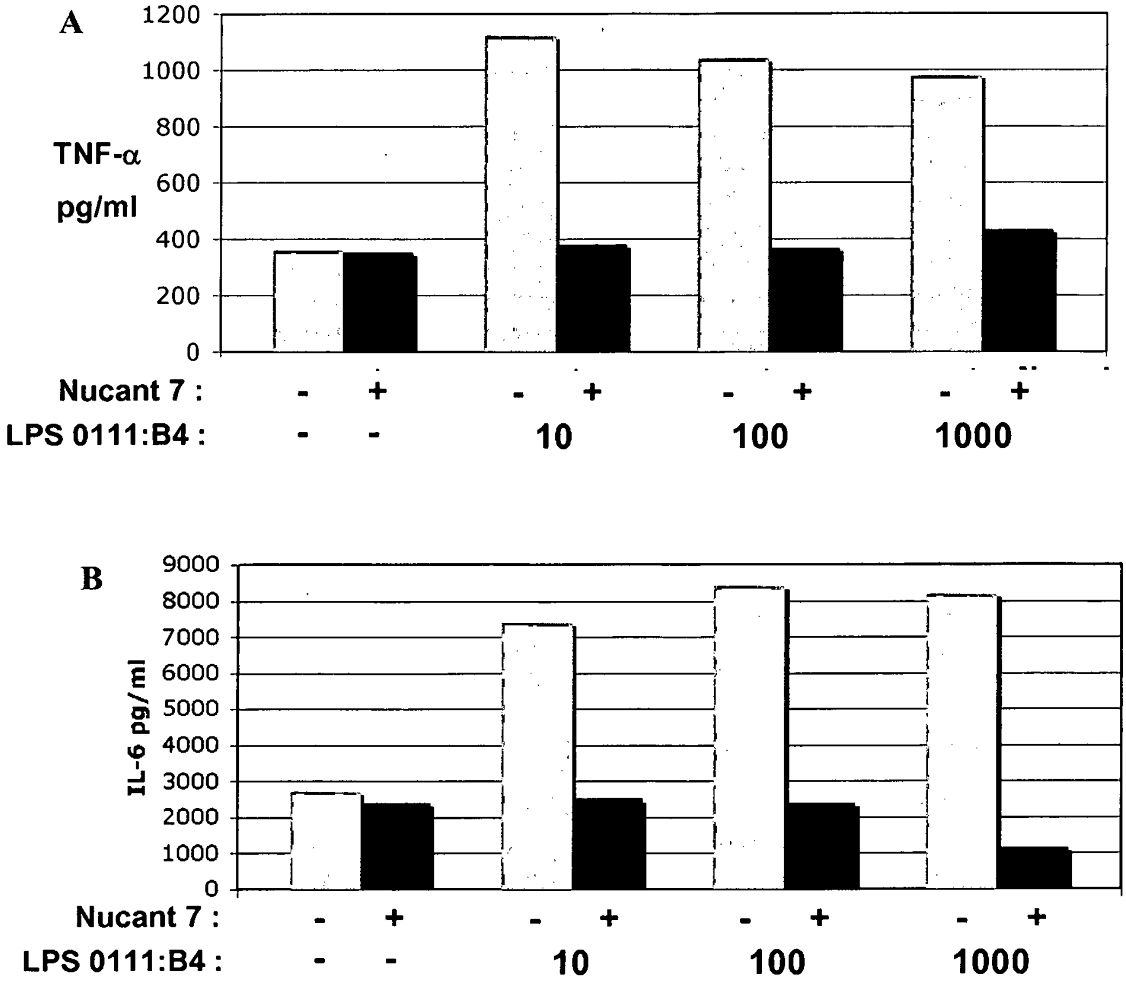
A
1200
1000
800
600
400
200
0
TNF-α
pg/ml
Nucant 7 :
- + - + - + - +
LPS 0111:B4 :
- - 10 100 1000
B
9000
8000
7000
6000
5000
4000
3000
2000
1000
0
IL-6 pg/ml
Nucant 7 :
- + - + - + - +
LPS 0111:B4 :
- - 10 100 1000

Figure 7

A

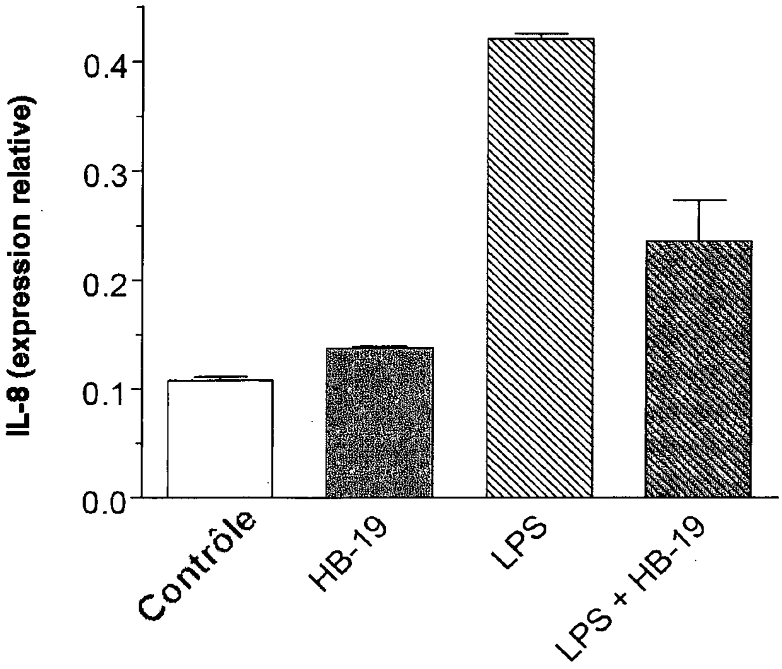
IL-8 (expression relative)
0.4
0.3
0.2
0.1
0.0
Contrôle
HB-19
LPS
LPS + HB-19

B

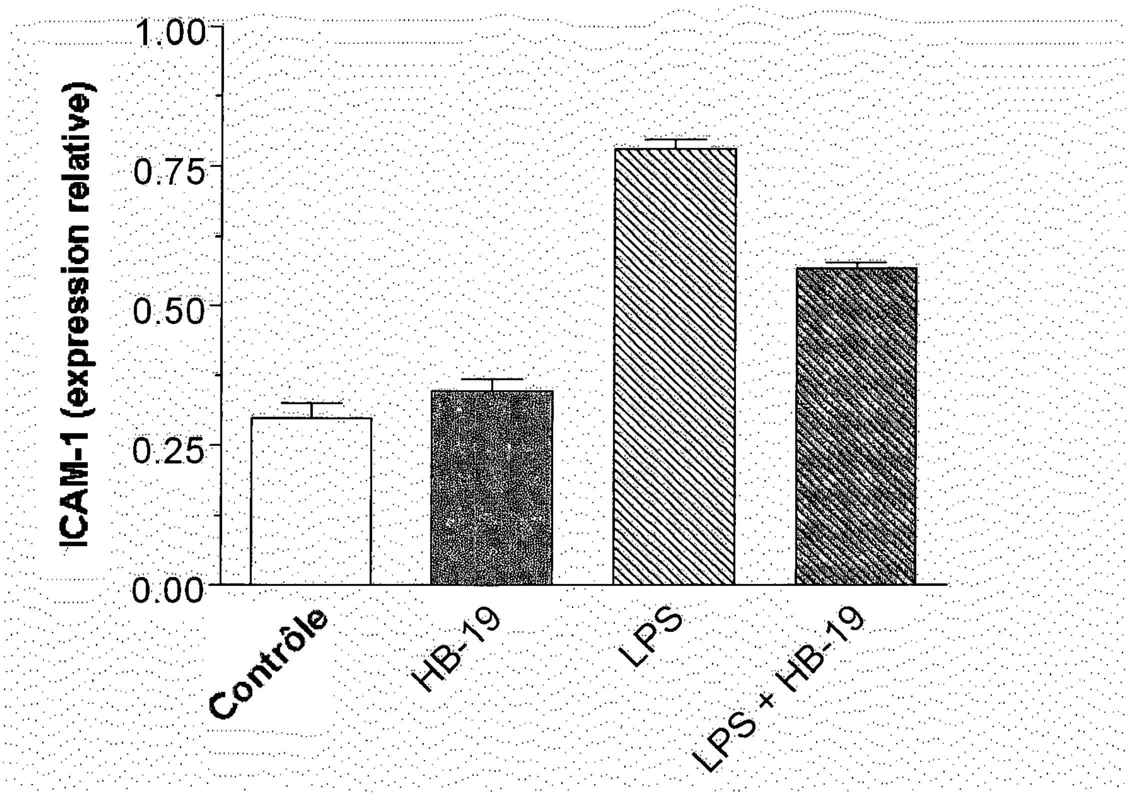
ICAM-1 (expression relative)
1.00
0.75
0.50
0.25
0.00
Contrôle
HB-19
LPS
LPS + HB-19

Figure 8

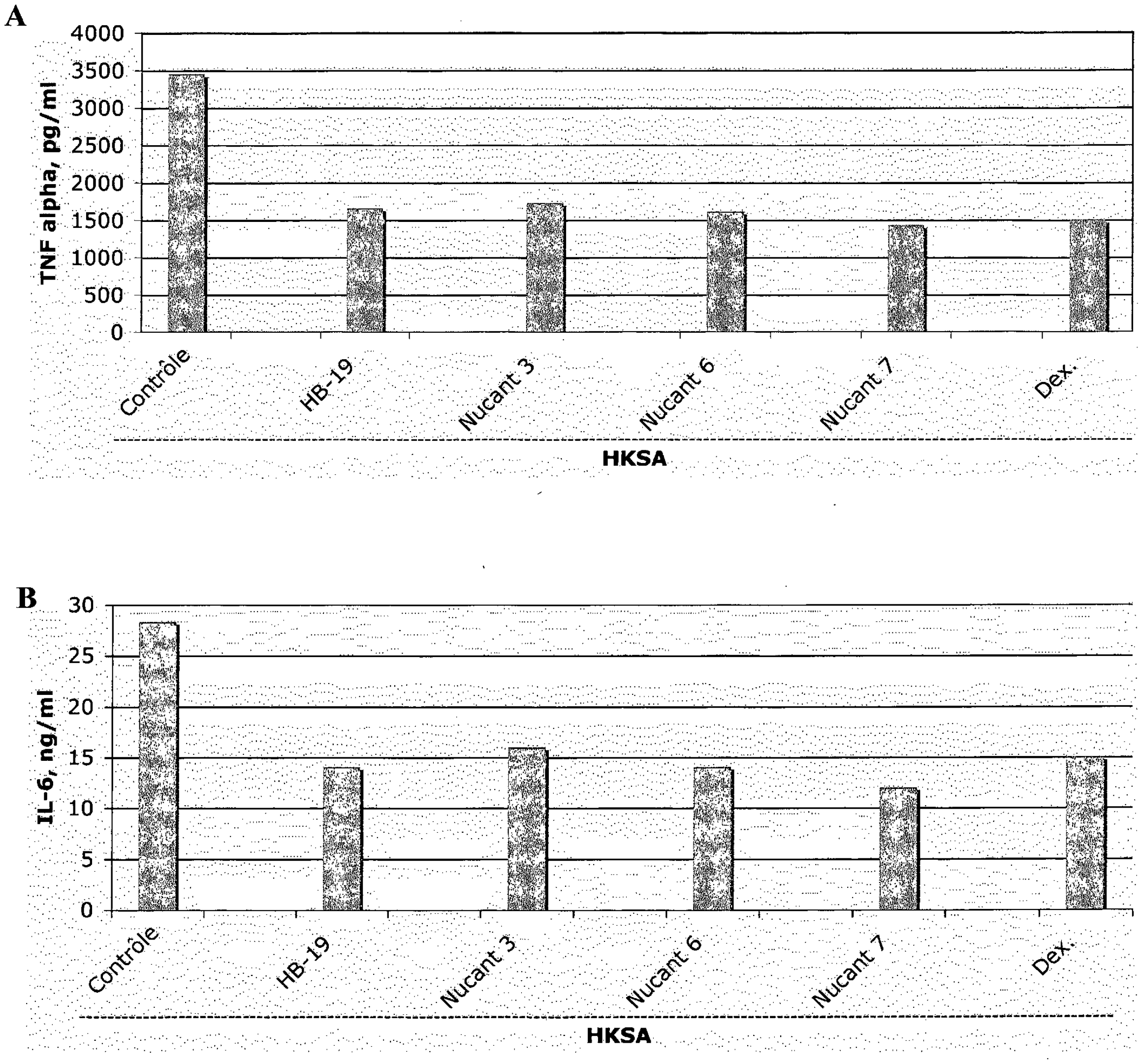
A
TNF alpha, pg/ml
4000
3500
3000
2500
2000
1500
1000
500
0
Contrôle
HB-19
Nucant 3
Nucant 6
Nucant 7
Dex.
HKSA
B
IL-6, ng/ml
30
25
20
15
10
5
0
Contrôle
HB-19
Nucant 3
Nucant 6
Nucant 7
Dex.
HKSA

Figure 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2007000730 W
- FR 0603813


### Littérature non-brevet citée dans la description


- **NISOLE et al.** *Exp. Cell Res,* 2002
- **NISOLE et al.** *J. Biol. Chem,* 2002
- **HOVANESSIAN, A.G. ; PUVION-DUTILLEUL, F. ; NISOLE, S. ; SVAB, J. ; PERRET, E. ; DENG, J. S. ; KRUST, B.** The cell-surface-expressed nucleolin is associated with the actin cytoskeleton. *Exp. Cell Res.,* 2000, vol. 261, 312-328
- **NISOLE, S. ; KRUST, B. ; CALLEBAUT, C. ; GUICHARD, G. ; MULLER, S. ; BRIAND, J. P. ; HOVANESSIAN, A. G.** The anti-HIV composé HB-19 forms a complex with the cell-surface-expressed nucleolin independent of heparan sulfate proteoglycans. *J. Biol. Chem.,* 1999, vol. 274, 27875-27884
- **GINISTY, H. ; SICARD, H. ; ROGER, B. ; BOUVET, P.** Structure and functions of nucleolin. *J. Cell Science,* 1999, vol. 112, 761-772
- **SRIVASTAVA, M. ; POLLARD, H. B.** Molecular dissection of nucleolin's role in growth and cell proliferation: new insights. *FASEB J.,* 1999, vol. 13, 1911-1922
- **NISOLE, S. ; KRUST, B. ; HOVANESSIAN, A. G.** Anchorage of HIV on permissive cells leads to coaggregation of viral particles with surface nucleolin at membrane raft microdomains. *Exp. Cell Res.,* 2002, vol. 276, 155-173
- **CALLEBAUT, C. ; JACOTOT, E. ; KRUST, B. ; GUICHAR, G. ; BLANCO, J. ; SVAB, J. ; MULLER, S. ; BRIAND, J. P. ; HOVANESSIAN, A. G.** Composé TASP inhibitors of HIV entry bind specifically to a 95-kDa cell surface protein. *J. Biol. Chem.,* 1997, vol. 272, 7159-7166
- **CALLEBAUT, C. ; BLANCO, J. ; BENKIRANE, N. ; KRUST, B. ; JACOTOT, E. ; GUICHARD, G. ; SEDDIKI, N. ; SVAB, J. ; DAM, E. ; MULLER, S.** Identification of V3 loop-binding proteins as potential receptors implicated in the binding of HIV particles to CD4(+) cells. *J. Biol. Chem.,* 1998, vol. 273, 21988-2199
- **SAID, A. E. ; KRUST, B. ; NISOLE, S. ; BRIAND, J. P. ; HOVANESSIAN, A. G.** The anti-HIV cytokine midkine binds the cell surface-expressed nucleolin as a low affinity receptor. *J. Biol. Chem.,* 2002, vol. 277, 37492-37502
- **SAID, E. A. ; COURTY, J. ; SVAB, J. ; DELBÉ, J. ; KRUST, B. ; HOVANESSIAN, A. G.** Pleiotrophin inhibits HIV infection by binding the cell surface-expressed nucleolin. *FEBS J.,* 2005, vol. 272, 4646-4659
- **LEGRAND, D. ; VIGIE, K. ; SAID, E. A. ; ELASS, E. ; MASSON, M. ; SLOMIANNY, M. C. ; CARPENTIER, M. ; BRIAND, J. P. ; MAZURIER, J. ; HOVANESSIAN, A. G.** Surface nucleolin participates in both the binding and endocytosis of lactoferrin in target cells. *Eur. J. Biochem.,* 2004, vol. 271, 303-317
- **NISOLE, S. ; KRUST, B. ; DAM, E. ; BLANCO, A. ; SEDDIKI, N. ; LOAEC, S. ; CALLEBAUT, C. ; GUICHARD, G. ; MULLER, S. ; BRIAND, J. P.** The HB-19 composé 5[Kpsi(CH2N)PR]-TASP inhibits attachment of T lymophocyte- and macrophage-tropic HIV to permissive cells. *AIDS Res. Hum. Retroviruses,* 2000, vol. 16, 237-249
- **NISOLE, S. ; SAID, E. A. ; MISCHE, C. ; PREVOST, M. C. ; KRUST, B. ; BOUVET, P. ; BIANCO, A. ; BRIAND, J. P. ; HOVANESSIAN, A. G.** The anti-HIV pentameric composé HB-19 binds the C-terminal end of nucleolin and prevents anchorage of virus particles in the plasma membrane of target cells. *J. Biol. Chem.,* 2002, vol. 277, 20877-20886
- **HUANG Y ; SHI H ; ZHOU H ; SONG X ; YUAN S ; LUO Y.** The angiogenesis function of nucleolin is mediated by vascular endothelial growth factor and nonmuscle myosin. *Blood,* 2006, vol. 107, 3564-3571
- **SEKO Y ; COLE S ; KASPRZAK W ; SHAPIRO BA ; RAGHEB JA.** The role of cytokine mRNA stability in the pathogenesis of autoimmune disease. *Autoimmun Rev,* 2006, vol. 5, 299-305
- **ROT A.** Neutrophil attractant/activation protein-1 (intrleukin-8) induces in vitro neutrophil migration by haptotactic mechanism. *Eur J Immunol,* 1993, vol. 23, 303-06
- **ELASS E ; MASSON M ; MAZURIER J ; LEGRAND D.** Lactoferrin inhibits the lipposaccharaide-induced expression and proteoglycan-binding ability of IL-8 in human entothelial cells. *Infect Immun,* 2002, vol. 70, 1860-66

- **KEVIL CG ; PATEL RP ; BULLARD DC.** Essential role of ICAM-1 in mediating monocyte adhesion to aortic endothelial cells. *Am J Physiol Cell Physiol,* 2001, vol. 281, C1442-47
- **BUCKLIN SE ; SILVERSTEIN R ; MORRISON DC.** An interleukin-6-induced acute-phase response does not confer protection against lipopolysaccharide lethality. *Infect Immun,* 1993, vol. 61, 3184-89
- **KARIMA R ; MATSUMOTO S ; HIGASHI H ; MATSUSHIMA K.** The molecular pathogenesis of endotoxic shock and organ failure. *Mol Med Today,* 1999, vol. 5, 123-32
- **ZANOTTI S ; KULMAR A ; KUMAR A.** Cytokine modulation in sepsis and septic shock. *Expert Opin Investig Drugs,* 2002, vol. 11, 1061-75
- **ALTER, P ; HOESCHEN, J. ; RITTER, M. ; MAISCH, B.** Usefulness of cytokines interleukin-6 and interleukin-2R concentrations in diagnosing active infective endocarditis involving native valves. *Am. J. Cardiol.,* 2002, vol. 89, 1400-1404
- **SHUN, C.T. ; LU, S.Y. ; YEH, C.Y. ; CHAING, C.P. ; CHIA, J.S. ; CHEN, J.Y.** Glucosyltransferases of viridans streptococci are modulins of interleukin-6 induction in infective endocarditis. *Infec. Immun.,* 2005, vol. 73, 3261-3270
- **KANNAN K ; ORTMANN RA ; KIMPEL D.** Animal models of rheumatoid arthritis and their relevance to human disease. *Pathophysiology,* 2005, vol. 12, 167-81
- **FOURNEL, S. et al.** C3-symmetric peptide scaffolds are functional mimetics of trimeric CD40L. *Nat Chem Biol,* 2005, vol. 1, 377-382